(19)

Europäisches
Patentamt
European
Patent Office
Office européen
des brevets

(11) **EP 4 704 104 A1**

(12) **EUROPEAN PATENT APPLICATION**

(43) Date of publication:
**04.03.2026 Bulletin 2026/10**

(21) Application number: **24197325.4**

(22) Date of filing: **29.08.2024**

(51) International Patent Classification (IPC):
**G16H 30/40** (2018.01)      **G16H 50/20** (2018.01)
**G16H 50/30** (2018.01)

(52) Cooperative Patent Classification (CPC):
**G16H 30/40; G16H 50/20; G16H 50/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **3Shape A/S**
**1060 Copenhagen K (DK)**

(72) Inventor: **AVRAMOSKI, Kiril**
**1060 Copenhagen K (DK)**

(74) Representative: **Zacco Denmark A/S**
**Arne Jacobsens Allé 15**
**2300 Copenhagen S (DK)**

(54) **METHOD FOR DETERMINING AN EPICENTER OF DENTAL CONDITIONS**

(57)    This disclosure relates to a computer-implemented method comprising the steps of obtaining a three-dimensional (3D) model of a dental site, the 3D model comprising a plurality of data points describing a surface of the dental site in 3D space; estimating a presence of one or more dental conditions defining one or more inspection sites in the 3D model, wherein each inspection site comprises one or more of the plurality of data points; determining an aggregated severity level of the one or more dental conditions for each of the one or more of the plurality of data points, thereby determining one or more aggregated severity levels for each inspection site; and determining an epicenter of the one or more aggregated severity levels of each inspection site by determining a maximum aggregated severity level of each inspection site and associating the one or more data points of the maximum aggregated severity level with a location of the epicenter.

Fig. 8b

EP 4 704 104 A1

## Description

### Technical field

The present disclosure relates to the field of dental diagnostics and, in particular, to a computer-implemented method and system for determining an epicenter of one or more dental conditions of a dental site.

### Background

Nowadays, most dental health conditions are preventable, and many of them are even treatable if detected in the early stages of their development. The detection of a health condition of a dental site of a patient requires an examination of the dental site by a dental practitioner, which may be carried out during a clinical visit of the patient in a dental studio. During the clinical visit, the dental practitioner may look for possible problem areas of the dental site of the patient to assess the overall health status of the patient's dental site, the latter including at least the patient's dentition and gingiva. Typically, the dental practitioner looks for possible cavities, tooth cracks, tooth wear or other conditions in the patient's teeth, then they may review existing restorations, and finally they may proceed with examining the health status of the patient's gingiva.

Additional diagnostic tools may be used by dental practitioners to support and optimize the examination process. Among these tools, digital dentistry is certainly one of the most widely used techniques nowadays due to its advantages over non-digital techniques. Digital dentistry enables dental practitioners to perform a scan of the patient's dental site by means of an intraoral scanner which can acquire scan data relative to the dental site. The scan data may be used to generate a three-dimensional (3D) digital representation of the dental site of the patient, also known as a 3D model of the dental site, which may be input to a number of software solutions, including, but not limited to, patient monitoring systems, and dental software systems configured to estimate a presence of possible problem areas in the patient's dental site, namely automated diagnostic tools. Said automated diagnostic tools offer solutions to dental practitioners to smoothly detect, classify and/or quantify the severity of the conditions present in the patient's dental site.

A continuous effort in digital dentistry is on improving automated diagnostic tools for a variety of dental conditions of the patient's dental site, with the goal of optimizing both the diagnostic process and the conveyance of information about the detected dental conditions from the dental practitioner to the patient. Currently existing solutions enable the dental practitioner to visualize the output of a diagnostic tool for a specific dental condition on a display, e.g. the display of a computer in the dental studio used by the dental practitioner during the clinical visit. Typically, a graphical representation of the output is displayed on a rendering of the 3D model of the patient's dental site. For example, the graphical representation of the output of a diagnostic tool for a specific dental condition may be a color or a heatmap overlaid on the rendering of the 3D model of the dental site, or the graphical representation may be a pointer to each detected problem area of the 3D model of the dental site. However, current solutions only allow the dental practitioner to visualize the graphical representation of one dental condition at a time on the 3D model of the dental site, and accordingly to assess a presence and/or a severity of one dental condition at a time. If the dental practitioner wants to examine more than one dental condition, e.g. caries, tooth crack and plaque, they have to manually select the dental condition they want to inspect, and manually switch between the visualization of the graphical representation of different dental conditions on the displayed 3D model of the dental site.

Current diagnostic tools do not enable visualizing the graphical representation of all the detected dental conditions on a same rendering of the 3D model of the dental site. Accordingly, existing diagnostic tools suffer from dispersing information relative to the overall health status of the dental site, and dental practitioners do not have any tool to objectively determine the locations of the dental site which are affected by more dental conditions. Furthermore, manually switching between the graphical representation of different dental conditions may cause the dental practitioner to miss problem areas of the dental site. Another drawback of existing diagnostic tools is that it may be challenging for the dental practitioner to convey to the patient the information that one or more locations of their dental site necessitate a treatment plan due to their health status, as switching between the graphical representation of different dental conditions does not convey to the patient a clear and effective visualization of the overall health status of their dental site.

Given the importance of an early detection and/or treatment of any existing dental condition in the patient's dental site, it is crucial to develop a diagnostic tool enabling dental practitioners to easily and efficiently assess the overall health status of the patient's dental site at once. A diagnostic tool providing dental practitioners with an effective guide to carry out the examination of the patient's dental site may also be time-saving both for practitioners and for patients. In addition to this, there is a need to develop improved diagnostic tools to optimize the communication between the dental practitioner and the patient.

### Summary

It is one aspect of the present disclosure to provide a computer-implemented method that overcomes the above-mentioned disadvantages. Accordingly, in one aspect there is disclosed herein a computer-implemented method comprising the steps of:

- obtaining a 3D model of a dental site, the 3D model comprising a plurality of data points collectively describing a surface of the dental site in 3D space;
- estimating a presence of one or more dental conditions defining one or more inspection sites in the 3D model, wherein each inspection site comprises one or more of the plurality of data points;
- determining an aggregated severity level of the one or more dental conditions for each of the one or more of the plurality of data points, thereby determining one or more aggregated severity levels for each inspection site; and
- determining an epicenter of the one or more aggregated severity levels of each inspection site by determining a maximum aggregated severity level of the inspection site and associating the one or more data points of the maximum aggregated severity level with a location of the epicenter.

Advantageously, the epicenter comprises quantified information relative to the aggregated severity of the one or more dental conditions, and it therefore localizes one or more specific areas of the dental site for which the overall health status is determined to be, locally or globally, the lowest, i.e. for which the health status is the worst. In other words, the epicenter localizes one or more specific areas of the dental site which necessitate the highest attention in an examination process carried out by a dental practitioner. Consequently, one or more locations of the dental site which necessitate careful inspection by the dental practitioner are accurately determined at once. The method disclosed herein significantly optimizes the diagnostic efficiency, as it may provide the dental practitioner with a clear and effective guide to perform an examination of the dental site. Said examination may be started from the epicenter of each inspection site, thereby enabling to prevent the development of further dental conditions in/around the epicenter and enabling to draw up a treatment plan for dental conditions at an advanced stage of their development.

The computer-implemented method may comprise obtaining a 3D model of a dental site. The 3D model may be a digital representation of a 3D geometry of the dental site, wherein the 3D geometry may describe intraoral features of the patient, including at least teeth and gingiva, but it may also comprise hard palate, soft palate and so forth.

The 3D model of the dental site may comprise a plurality of data points collectively describing a surface of the dental site in 3D space. The plurality of data points may be elements that the above-mentioned 3D geometry consists of, namely the elements that can describe a surface of the dental site in 3D space. In one example, the plurality of data points may be the plurality of 3D points of a point cloud, wherein each point in the point cloud is described by a set of Cartesian coordinates $(x, y, z)$. In another example, the plurality of data points may be a plurality of facets of a mesh parametrizing the surface of the dental site in 3D space. Each of said facets may be a triangle in the case where the mesh is a triangle mesh, or another polygon in the case where the mesh is a polygonal mesh, or any other geometrical shape suitable for representing a 3D model. In yet another example, the plurality of data points may be a plurality of vertices comprised in a mesh, wherein the mesh may be a triangle mesh or any other polygonal mesh suitable for describing the surface of the dental site in 3D space.

As the computer-implemented method disclosed herein may be based on using a trained learning model, it is an advantage that the 3D model comprises the plurality of data points, as the plurality of data points enables the 3D model to be processed using the trained learning model. Therefore, the plurality of data points ensures that a discretized input which is suitable for the trained learning model is provided to said training model. Advantageously, this allows efficient processing of the obtained 3D model of the dental site. Further explanation of the usage of a trained learning model will be elaborated on throughout the description.

The computer-implemented method may further comprise estimating a presence of one or more dental conditions defining one or more inspection sites in the 3D model. Each inspection site may comprise one or more of the plurality of data points. Estimating a presence of one or more dental conditions may comprise processing the plurality of data points using a trained learning model to estimate the presence of the one or more dental conditions. The trained learning model may be a pre-trained machine learning model for each of the one or more dental conditions, or it may be a pre-trained machined learning model for all of the one or more dental conditions. The pre-trained machine learning model may have been trained on training data comprising data points similar to the plurality of data points, the training data further comprising quantified local information about features of each data point in the training data.

Advantageously, the processing of the plurality of data points provides local information relative to the one or more dental conditions for the 3D model, enabling to accurately localize one or more regions of the 3D model for which the presence of at least one dental condition is estimated, i.e. the one or more inspection sites of the 3D model. A further advantage of determining local information relative to the one or more dental conditions is that only the one or more data points comprised in each inspection site are further processed, thereby reducing the computational time of the computer-implemented method.

The computer-implemented method may further comprise determining an aggregated severity level of the one or more dental conditions for each of the one or more of the plurality of data points, thereby determining one or more aggregated severity levels for each inspection site. The aggregated severity level of the one or more dental conditions may be a sum or a weighted sum of severity levels, wherein each severity level may be determined for a specific dental condition. For each data point, the severity level of each dental condition may be a score quantifying the severity of that specific dental condition at the location of that data point and said score may be defined in a suitable manner based on clinical aspects of the dental condition. Advantageously, determining the aggregated severity level for each data point in each inspection site ensures that an overall dental health condition is locally quantified. Further, determining the aggregated severity level for each data point in each inspection site allows to determine one or more epicenters of the dental conditions in the 3D model, i.e. the one or more regions of the dental site which necessitate the highest attention by the dental practitioner. This is not possible with current diagnostic tools, which typically provide a quantification of the severity of a dental condition at a time and do not enable to quantify the overall health status of the dental site.

The computer-implemented method may further comprise determining an epicenter of the one or more dental conditions of each inspection site by determining a maximum aggregated severity level of each inspection site and associating the one or more data points of the maximum aggregated severity level with a location of the epicenter. Thus, one or more epicenters of the dental conditions may be determined for the 3D model of the dental site. In an alternative embodiment, the method may comprise determining only one epicenter of the dental conditions by determining a global maximum aggregated severity level among the aggregated severity levels determined for all of the inspection sites of the 3D model. In other words, the method disclosed herein may comprise determining at least one epicenter of the dental conditions. The one or more aggregated severity levels determined for each inspection site may vary over a broad range, for example in the case where the inspection site comprises many data points. This may result in the dispersion of the information relative to the overall health status of the dental site. It is therefore an advantage that one or more epicenters are determined, as this accurately localizes one or more areas of the 3D model of the dental site for which the overall health status is determined to be, locally or globally, the lowest/worst. In other words, determining at least one epicenter enables to localize one or more areas of the dental site which necessitates the highest attention by the dental practitioner. Advantageously, the determined one or more epicenters provide the dental practitioner with an effective guide to perform an examination of the dental site of the patient, as the examination may be started by the locations associated with the one or more data points of each epicenter where the challenges associated with detected dental conditions are the highest.

The computer-implemented method may further comprise associating the determined one or more aggregated severity levels with the plurality of data points of the 3D model and rendering the 3D model on a graphical user interface (GUI) with a graphical representation of the one or more aggregated severity levels. Each determined aggregated severity level maps to one data point of the 3D model, such that each data point of the 3D model is associated with quantified local information relative to the severity of the one or more dental condition. This severity mapping enables to generate a graphical representation of the one or more aggregated severity levels. Advantageously, the graphical representation enables the dental practitioner to identify at once the one or more locations of the dental site which necessitate careful inspection due to their overall health condition.

The graphical representation of the one or more aggregated severity levels may be displayed as an indication on the 3D model. The indication may be intended as a texture, a pointer, a symbol, or the like indicating the areas of the dental site affected by the one or more dental conditions. Advantageously, the indication may be such that a contrast is generated between the rendered 3D model and the one or more aggregated severity levels, thereby highlighting in a clear way the locations of the 3D model for which the presence of at least one dental condition is estimated. This ensures that the dental practitioner is assisted and guided in performing the examination process of the dental site of the patient, whereby the time needed to perform said examination is significantly reduced with respect to the case where the dental practitioner performs the in-mouth examination without such a graphical representation. In practice, this fast examination could have not been achieved if the dental practitioner was not provided with the indication of the areas of the dental site which are highly affected by the one or more dental conditions, i.e. with the indication of the inspection sites and the corresponding epicenter(s).

The indication may be a color different than a rendering color of the 3D model, and an intensity of the color may decrease with a distance from the estimated epicenter of each inspection site. Therefore, the larger the intensity of the color in a region of the rendering of the 3D model, the larger the severity of the one or more dental conditions determined in that region. This is an advantage, as the dental practitioner may start the examination of the patient's dental site by inspecting those locations of the dental site which are displayed with the largest color intensity on the 3D model. Said locations may be the ones where a dental condition at an advanced stage of its development is present, or they may be the ones where more dental conditions are present. In both cases, the intensity of the color enables to draw the attention of the dental practitioner to the locations of the dental site which may necessitate a treatment plan and/or a plan to prevent the further development of the one or more dental conditions. Then, the dental practitioner may proceed with the in-mouth examination by moving to areas of the dental site which are displayed with progressively decreasing intensity. In other words, the intensity of the color generates a hierarchy of a clinical significance of one or more regions of the 3D model, thereby providing the dental practitioner with a guide to perform the examination process which makes the diagnostic process quick and effective.

The indication may be a pattern, and a density of the pattern may decrease with a distance from the estimated epicenter of each inspection site. Therefore, the larger the density of the pattern in a region of the rendering of the 3D model, the more severe the determined overall health status in that region. This is an advantage, as the dental practitioner may start the examination of the patient's dental site by inspecting the locations of the dental site which are displayed with the highest pattern density on the 3D model. Said locations may be the ones where a dental condition at an advanced stage of its development is present, or they may be the ones where more dental conditions are present. In both cases, the density of the pattern enables to draw the attention of the dental practitioner to the locations of the dental site which may necessitate a treatment plan and/or a plan to prevent the further development of the one or more dental conditions. Then, the dental practitioner may proceed with the in-mouth examination by moving to areas of the dental site which are displayed with progressively decreasing density. In other words, the density of the pattern generates a hierarchy of a clinical significance of one or more regions of the 3D model, thereby providing the dental practitioner with a guide which makes the diagnostic process quick and effective.

The method may further comprise calculating the distance using a Euclidean distance measure. The Euclidean distance measure is advantageous when the plurality of data points comprises a point cloud, as each point of the point cloud is described by Cartesian coordinates $(x,y,z)$ in Euclidean space.

The method may further comprise calculating the distance using a geodesic distance measure. The geodesic distance measure is advantageous when the plurality of data points comprises a triangle mesh comprising facets and vertices, as the facets and vertices of the mesh comprise information about a local curvature of the surface of the dental site.

The method may further comprise calculating the distance using a facet distance measure. The facet distance measure may be intended as a measure of the minimum number of steps to go from a facet to another facet only moving through neighboring facets. Therefore, the facet distance measure is advantageous when the plurality of data points comprises a plurality of facets comprised in a mesh describing the surface of the dental site in 3D space.

The one or more dental conditions may comprise one or more of caries, gum recession, tooth wear, plaque, gum inflammation, and tooth crack. Those are among the most common dental health conditions which can be detected at early stages of their development. However, the one or more dental conditions may comprise any other dental condition which is not mentioned above and which may be of interest in the examination process of the patient's dental site.

Estimating the presence of the one or more dental conditions may comprise processing the plurality of data points using a trained machine learning model for each of the one or more dental conditions. Advantageously, the trained machine learning model performs a local processing of the 3D model, and therefore outputs a local estimation of the presence of the one or more dental conditions in the 3D model. This improves the accuracy of the localization of one or more areas of the 3D model where one or more dental conditions are present.

The trained machine learning model for each dental condition may output for each data point a severity level of each dental condition, and the severity level may be associated with a number on a predefined scale of severity of each dental condition. This ensures that the severity of each dental condition is locally quantified in the 3D model.

The predefined scale of severity of each dental condition may comprise a minimum number which is indicative of an absent dental condition. Advantageously, one or more data points of the 3D model for which the minimum number on the scale of severity is determined are not further processed, reducing the computational complexity and computational execution time of the method disclosed herein. In other words, the method comprises processing the data points which are associated with at least a number which is larger than the minimum number.

The minimum number may be zero. Advantageously, as some steps of the method may involve mathematical manipulation of the severity levels, the number zero ensures that no contribution is given by the minimum severity level in said mathematical steps, e.g. in steps involving sums or multiplications such as the calculation of the aggregated severity level for each data point. Furthermore, from an imaging perspective, the number zero corresponds to an absence of a color and/or pattern, e.g. in a red, green, blue (RGB) scale, or in a cyan, magenta, yellow, key/black (CMYK) scale, or in a dots per inch (DPI) scale and so forth. Hence, it is an advantage that when the number zero is associated with a data point, that data point is displayed on the 3D model with the same color of the rendering color of the dental site.

Determining the aggregated severity level of the one or more dental conditions for each data point comprises determining the severity level of each dental condition for each data point, and for each data point aggregating the determined severity level of each dental condition. Advantageously, the overall health condition is locally determined in the 3D model, i.e. for each data point in each inspection site, based on a quantified severity of each dental condition determined for that data point. This significantly improves the accuracy of the localization of the regions of the 3D model which are affected by the one or more dental conditions.

Aggregating the determined severity level of each dental condition may comprise summing the determined severity level of each dental condition. Advantageously, summing the determined severity level of each dental condition may reduce the computational cost and time of the method. Furthermore, as the sum assigns equal significance to each dental condition it prevents any dental condition from being overlooked, providing the dental practitioner with an objective and unbiased estimation of the presence of the one or more dental conditions in the dental site.

Aggregating the determined severity level of each dental condition may comprise determining a weighted sum of the determined severity level of each dental condition. Advantageously, the weighted sum ensures that each dental condition properly contributes to the quantification of the overall health status of each data point, e.g. based on realistic clinical features of that dental condition.

Determining the weighted sum may further comprise assigning a weight to the determined severity level of each dental condition based on a clinical significance of each dental condition. This ensures that dental conditions which are considered more important from a clinical perspective, e.g. because their stage of development advances quickly and/or because they may cause a development of other dental conditions, contribute more to the aggregated severity level than dental conditions which are instead categorized as less important with respect to a clinical standard. Advantageously, the weighted sum enables to determine a realistic overall health status of the dental site of the patient.

The plurality of data points may comprise a point cloud. The 3D model of the dental site may be generated upon acquiring data relative to the dental site by means of a 3D intraoral scanner, which may capture and/or generate a point cloud representing the surface of the dental site. It is therefore an advantage that the computer-implemented method disclosed herein is configured to be performed upon obtaining a 3D model comprising a point cloud.

The plurality of data points may comprise a plurality of facets of a triangle mesh. The 3D model of the dental site may be generated upon acquiring data relative to the dental site by means of a 3D intraoral scanner which may process the acquired data to generate a triangle mesh representing the surface of the dental site in 3D. Said triangle mesh may comprise a plurality of facets, and it is therefore an advantage that the computer-implemented method disclosed herein is suitable to be performed upon obtaining a 3D model comprising a plurality of facets.

The plurality of data points may comprise a plurality of vertices of a triangle mesh. The 3D model of the dental site may be generated upon acquiring data relative to the dental site by means of a 3D intraoral scanner, which may process the acquired data relative to generate a triangle mesh representing the surface of the dental site in 3D. Said triangle mesh may comprise a plurality of vertices, and it is therefore an advantage that the computer-implemented method disclosed herein is suitable to be performed by obtaining a 3D model comprising a plurality of vertices.

The computer-implemented method may further comprise smoothing the one or more aggregated severity levels determined for each inspection site, whereby one or more smoothed aggregated severity levels may be determined for each inspection site. The aggregated severity level may change abruptly when moving from a data point to a neighboring data point in the same inspection site or when moving between adjacent inspection sites. These inevitable discontinuities in each inspection site may cause the generation of an unclear graphical representation of the one or more aggregated severity levels, e.g. the boundaries of said locations may appear as edgy. Advantageously, the smoothing step ensures that the aggregated severity level smoothly changes when moving between neighboring data points, improving the graphical representation of the locations of the 3D model for which at least one dental condition is determined and facilitating the diagnostic process for the dental practitioner.

The one or more smoothed aggregated severity levels determined for each inspection site may linearly or exponentially decrease with the distance from the epicenter determined for each inspection site. The linear behavior of the smoothed aggregated severity levels ensures that the aggregated severity levels of each inspection site decrease at a constant rate when moving away from the epicenter of that inspection site. This may be advantageous to provide the dental practitioner with an indication of all the data points for which at least one dental condition is determined, rather than enhancing the epicenter of each inspection site. On the other hand, the exponential behavior ensures that the one or more aggregated severity levels of each inspection site rapidly fall off when moving away from the epicenter of each inspection site. This may be advantageous to provide the dental practitioner with an indication of the regions of the dental site which are affected by more severe dental conditions than others, i.e. it ensures that the epicenter of each inspection site stands out in the graphical representation of the one or more aggregated severity levels.

The computer-implemented method may comprise determining a global epicenter of the one or more dental conditions of the 3D model by determining a global maximum aggregated severity level among the one or more aggregated severity levels determined for the one or more inspection sites, and associating the one or more data points of the global maximum aggregated severity level with a location of the global epicenter. Advantageously, a region of the 3D model for which the overall health status is determined to be, globally, the lowest, i.e. the worst, is accurately determined. In other words, the global epicenter is the region of the dental site which necessitates, globally, the highest attention by the dental practitioner. Thus, the dental practitioner may promptly identify the location of the 3D model which necessitates particular attention and start the dental examination from the location of the global epicenter.

The computer-implemented method may comprise normalizing the one or more aggregated severity levels of the one or more inspection sites with the determined global maximum aggregated severity level, whereby the normalized one or more aggregated severity levels of the one or more inspection sites may vary between 1 and 0, wherein 1 is the normalized aggregated severity level of the global epicenter of the 3D model. Thus, the normalization may generate an order of clinical significance of the epicenters determined for the one or more inspection sites of the 3D model, based on the aggregated severity of the epicenter of each inspection site relative to the aggregated severity of the global epicenter. Advantageously, this provides a clear and effective diagnostic guide to the dental practitioner, who accordingly may start the examination of the dental site from the location of the global epicenter and proceed with locations for which a progressively decreasing aggregated severity level of the one or more dental conditions is determined.

The method may further comprise generating an ordered list of the one or more dental conditions estimated for each inspection site and displaying the ordered list on the GUI upon receiving an input signal. Therefore, the dental practitioner may have an accurate indication of the one or more dental conditions estimated for each inspection site and perform an in-mouth examination of the one or more inspection sites according to the ordered list.

The method may further comprise determining an order of importance of the severity level of each of the one or more dental conditions estimated for each inspection site, wherein the order may be a descending order. Advantageously, the dental practitioner may perform the in-mouth examination of the inspection sites of the patient's dental site using as a guide the order of importance of the severity level determined for each dental conditions. For example, the dental practitioner may start inspecting the dental condition which is determined to be the most severe in the generated ordered list and proceed with progressively less severe dental conditions. Thus, using the 3D model combined severity mapping guidance significantly facilitates the examination of the patient's dental site.

The method may further comprise storing the ordered list generated for each inspection site and loading the ordered list upon receiving an input signal. Advantageously, the stored ordered list can be accessed at any time by the dental practitioner by inputting a signal, for instance to review the severity levels determined for each inspection site in a previous examination of the patient's dental site and compare them with the severity levels determined for the same inspection site during the clinical visit of the patient.

The ordered list may comprise the severity level of each of the one or more dental conditions estimated for each inspection site. This provides the dental practitioner with a clear quantification of the severity of each dental condition locally determined for each inspection site.

The input signal may comprise hovering or clicking on an inspection site on the displayed 3D model with the graphical representation of the one or more aggregated severity levels. This provides a simple and practical operation for the dental practitioner to load and visualize the ordered list for each inspection site.

In another aspect, there is disclosed herein a program product comprising instructions which, when executed by a computer, cause the computer to perform the method steps described herein. Thus, in practice the method disclosed herein can be executed by any computer system configured to read the computer program product disclosed herein. This is an advantage, as the instructions comprised in said computer program ensure that the execution of one or more steps of the method disclosed herein is automated.

In yet another aspect, there is disclosed herein a non-volatile computer-readable medium comprising instructions which, when executed by a computer, cause the computer to perform the method according to any of the disclosed embodiments. Thus, in practice the instructions to perform the method disclosed herein can be performed on any computer system configured to read the non-volatile computer-readable medium disclosed herein. This is an advantage, as said instructions can be retained also when the computer system is turned off, enabling the user to access the instructions and/or the data stored on the non-volatile computer-readable medium at any time and from any computer system configured to read the non-volatile computer-readable medium disclosed herein.

In yet another aspect, there is disclosed herein a system which may comprise an intraoral scanner. The system may further comprise a computer system. Said computer system may comprise a display, a communication interface, one or more processors, and one or more memories. The one or more memories may contain a program content executable by the one or more processors. The program content may further comprise executable instructions to obtain a 3D model of a dental site, wherein the 3D model may comprise a plurality of data points collectively describing a surface of the dental site in 3D space. The program content may further comprise executable instructions to estimate a presence of one or more dental conditions defining one or more inspection sites in the 3D model, wherein each inspection site comprises one or more of the plurality of data points. The program content may further comprise executable instructions to determine an aggregated severity level of the one or more dental conditions for each of the one or more of the plurality of data points, thereby determining one or more aggregated severity levels for each inspection site. The program content may further comprise executable instructions to determine an epicenter of the one or more aggregated severity levels of each inspection site by determining a maximum aggregated severity level of the inspection site and to associate the one or more data points of the maximum aggregated severity level with a location of the epicenter.

Advantageously, the intraoral scanner enables the dental practitioner to perform a scan of the dental site of a patient, and therefore to acquire scan data relative to said dental site. The data acquired by the intraoral scanner may be used to generate the 3D model of the dental site. It is a further advantage that the system disclosed herein enables to determine the location of the epicenter of each inspection site, as the latter localizes a specific area of that inspection site for which the overall health status is determined to be the most severe. Consequently, one or more locations of the dental site which necessitate careful inspection by a dental practitioner are accurately determined at once. The system disclosed herein significantly optimizes the diagnostic efficiency, as it provides the dental practitioner with a clear and effective guide to perform an examination of the dental site. Said examination may be started from the epicenter of each inspection site, thereby enabling to prevent the development of further dental conditions in/around the epicenter and enabling to draw up a treatment plan for dental conditions at an advanced stage of their development. It is yet another advantage of the system disclosed herein that the computer system comprises the display, as the 3D model with a graphical representation of the one or more aggregated severity levels determined for each inspection site may be displayed on the display. The dental practitioner may use the displayed graphical representation as a guide and/or support to perform the in-mouth examination of the dental site and assess its overall health status. Thus, the system disclosed herein significantly optimizes the diagnostic process.

The communication interface may be configured to enable the computer system to exchange data with one or more external devices and with one or more external networks. Thus, the communication interface enables the computer system to send and/or receive data from the intraoral scanner comprised in the system disclosed herein. This is an advantage, as the computer system may receive from the intraoral scanner scan data relative to the patient's dental site acquired by the intraoral scanner. Furthermore, the communication interface enables the computer system to receive and/or send data to an external network which may be configured to perform at least a part of the processing of the 3D model, thereby facilitating and speeding up the processing of the 3D model.

The one or more external networks may comprise at least one cloud network. Thus, a part of the processing of the 3D model may be performed by the at least one cloud network. Advantageously, the cloud network may be configured to handle heavy workloads efficiently, thereby reducing the computational load of the computer system and optimizing the processing of the 3D model.

The at least one cloud network may comprise executable instructions to process the plurality of data points using a trained machined learning model for each of the one or more dental conditions. Thus, the computer system may send the obtained 3D model of the dental site to the cloud network, and receive the output of the trained machine learning model of the cloud network by means of the communication interface, without the need of having said machine learning model stored on the one or more memories comprised in the computer system. This significantly saves memory space of the computer system, and it also speeds up the processing of the plurality of data points.

## Brief description of the drawings

Aspects of the present disclosure may be best understood from the following detailed description alongside the following drawings. The drawings are schematic and simplified for the sake of clarity, and they are intended to show details to improve the understanding of the claims, whereas other details may be left out. Throughout the description and the drawings, the same reference numbers are used for identical or corresponding parts. The individual features of each aspect may each be combined with any or all features of the other aspects. These and other aspects, features and/or technical effects will be apparent from and elucidated with reference to the illustrations described hereinafter in which:

Figure 1 illustrates a flowchart according to an embodiment of the computer-implemented method disclosed herein;

Figure 2 illustrates an example of dental scanning system according to the present disclosure;

Figures 3a and 3b illustrate example reference diagrams for describing an embodiment of a workflow of the computer-implemented method disclosed herein;

Figure 4 illustrates an example reference diagram for describing a trained machine learning model for a dental condition on which the computer-implemented method disclosed herein may be based;

Figures 5a and 5b illustrate example reference diagrams for describing an embodiment of a workflow of the computer-implemented method disclosed herein;

Figures 6a, 6b and 6c illustrate a flowchart according to an embodiment of the computer implemented method disclosed herein;

Figures 7a, 7b, 7c, 7d, 7e, 7f and 7g illustrate an example of computer-implemented smoothing method according to the present disclosure;

Figures 8a and 8b illustrate examples of a graphical representation on a graphical-user interface (GUI) of an output of the computer-implemented method disclosed herein;

Figure 9 illustrates an example of a visualization on a graphical-user interface (GUI) of an output of the computer-implemented method disclosed herein.

Figure 10 illustrates an embodiment of the computer system disclosed herein.

## Detailed description

The detailed description set forth below in connection with the appended drawings is intended as a description of various examples according to the disclosure. The detailed description includes details for the purpose of providing a thorough understanding of various concepts and examples covered throughout the description. However, it will be apparent to those skilled in the art that these concepts and examples may be practiced without the specific details mentioned or in combination with one or more examples described herein. Several examples of the devices, systems, mediums, programs and methods are described by various modules, components, steps, processes, algorithms, etc. Depending upon particular application, design constraints or other reasons, these elements may be implemented using electronic hardware, computer program, or any combination thereof. In the following several examples of the method and system described herein will be disclosed in more detail.

The solutions presented herein generally aim at improving the automatic determination of an overall health status of a dental site by determining a location of one or more epicenters of one or more dental conditions, including caries, cracks, recession, wear, plaque, or any other relevant restorative elements forming part of the dental site. The solutions presented herein further aim at guiding or improving the dental health assessment and treatment process by providing a diagnostic tool that ensures an efficient and objective process.

Digital information relative to a patient's dental site may be obtained during a scanning session, when a dental practitioner scans, by means of an intraoral scanner, the dental site within an oral cavity of the patient. The intraoral scanner may be configured to acquire surface information of the dental site. The intraoral scanner may employ a scanning principle such as triangulation-based scanning, confocal scanning, focus scanning, ultrasound scanning, stereo vision, structure from motion, optical coherent tomography OCT, or any other scanning principle. Detailed information relative to examples of intraoral scanners will be given in the following detailed description.

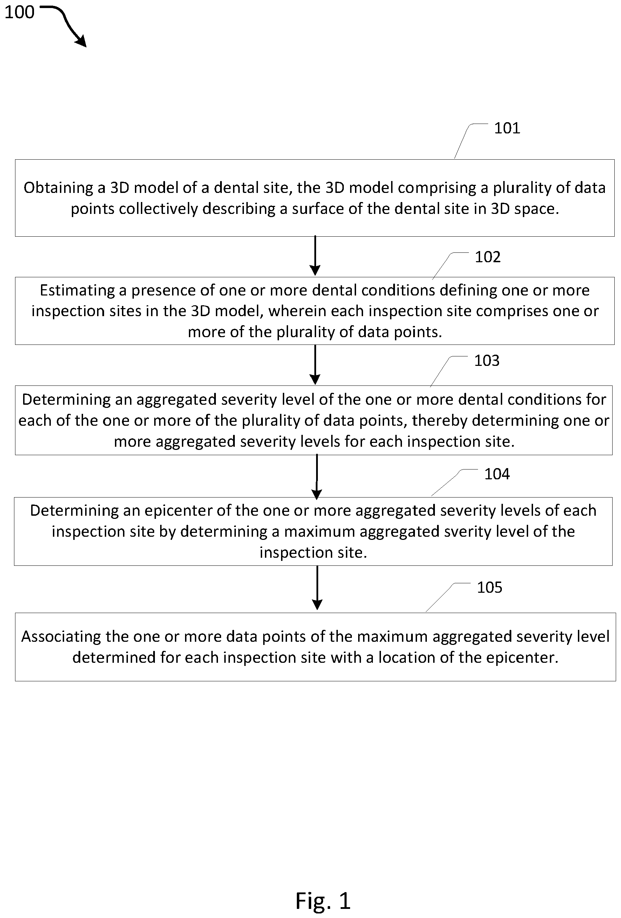

Figure 1 illustrates a flowchart 100 of an embodiment of the computer-implemented method disclosed herein. A three-dimensional (3D) model of a dental site is obtained in step 101. The dental site is part of an oral cavity of a patient, and it comprises at least the patient's teeth and gingiva, but it may also comprise soft palate, hard palate and any other part of the patient's oral cavity. The obtained 3D model may be generated during a clinical visit of the patient, upon scanning the dental site of the patient with an intraoral scanner. Alternatively, the 3D model may have been generated and stored during a previous clinical visit of the patient, and thus said 3D model of the dental site may be obtained upon loading the stored 3D model, e.g. from a memory where it has been stored during the previous scanning session. The 3D model may comprise a plurality of data points which collectively describe a surface of the dental site in 3D space. For example, the plurality of data points may comprise a point cloud, a polygon mesh, a voxel model, a triangulated point cloud, or any other method of storing information about a surface of the scanned dental site in 3D space, i.e. a 3D geometry of the dental site. In another example, the plurality of data points may comprise a triangulated mesh, wherein the triangulated mesh comprises a set of triangles which are connected by their common edges or vertices. In general, the plurality of data points comprised in the 3D model describes at least the 3D geometry of the surface of the patient's dental site.

In step 102, a presence of one or more dental conditions is estimated, thereby defining one or more inspection sites in the 3D model, wherein each inspection site comprises one or more of the plurality of data points. The one or more dental conditions may comprise one or more of caries, cracks, wear, plaque, recession, and any other dental condition which may be of interest for assessing the overall dental health of the patient. Estimating the presence of the one or more dental conditions may comprise processing the plurality of data points comprised in the 3D model using a trained machine learning model for each dental condition. For example, the presence of caries may be estimated by inputting each data point in a trained machine learning model for caries detection, the presence of cracks may be estimated by inputting each data point in a trained machine learning model for cracks detection, the presence of wear may be estimated by inputting each data point in a trained machine learning model for wear detection, and so forth for each dental condition. The trained machine learning model for each dental condition may be a pre-trained machine learning model that has been trained using a training data set comprising data points similar to the plurality of data points of the 3D model, wherein the training data set also comprises information about features of each data point of the training data set. For example, the features may be the presence or absence of caries for each data point in the training data set for the trained machine learning model for caries, and the features may be the presence or absence of cracks for each data point in the training data set for the trained machine learning model for tooth cracks, and so forth for each dental condition. The features may further or alternatively represent a severity label, and/or size, and/or area and so forth of each dental condition.

The trained machine learning model for a specific dental condition may output for each data point a severity level of that dental condition, wherein the severity level may be a score indicating the severity of that condition. For example, the severity level of the dental condition may be chosen from the group of no condition ($N$), initial condition ($I$), moderate condition ($M$) and/or severe condition ($S$). Alternatively, the severity level of the dental condition may be chosen between presence (P) of the condition and no presence (NP) of the condition. Further, each severity level may be associated with a number on a predefined scale of severity of each dental condition, such that each severity level quantifies the severity of that condition. The scale of severity of each dental condition may be defined in a suitable manner describing clinical aspects of that specific condition, i.e. different numbers may be comprised in the scale of severity of different dental conditions based on clinical features of each dental condition. The scale of severity may comprise a minimum number, e.g. the number zero, which is indicative of an absent dental condition. For example, the severity level $N$ may be associated with the number 0, the severity level $I$ may be associated with the number 1, the severity level $M$ may be associated with the number 3, and the severity level $S$ may be associated with the number 9, i.e. the mapping:

$$(N, I, M, S) \leftrightarrow (0, 1, 3, 9)$$

may hold for a specific dental condition. In another example, the severity level NP may be associated with the number 0, and the severity level P may be associated with the number 1, i.e. the mapping:

$$(NP, P) \leftrightarrow (0, 1)$$

may hold for a specific dental condition. The above-mentioned mappings are intended as examples, and any severity scale suitable to quantify the severity of a dental condition may be used. The one or more data points for which the presence of at least one dental condition is estimated by the trained machine learning model define one or more inspection sites of the 3D model. In particular, the one or more data points comprised in each inspection site are associated with at least one severity level of a dental condition which is different from the minimum severity level of that dental condition.

In step 103, an aggregated severity level of the one or more dental conditions for each of the data point in each inspection site is determined, thereby determining one or more aggregated severity levels for each inspection site. The aggregated severity level for each data point may be determined by summing the severity level of each dental condition determined for that data point. For example, referring to a same data point, a severity level of 3 may have been determined for caries, a severity level of 0 may have been determined for cracks, a severity level of 9 may have been determined for wear, a severity level of 3 may have been determined for recession, and a severity level of 1 may have been determined for plaque. In this example, the aggregated severity level of (3+0+9+3+1)=16 is determined for the data point.

The aggregated severity level for each data point may alternatively be determined by determining a weighted sum of the severity level determined for each dental condition. In this case, each dental condition contributes to the aggregated severity level with a weight which may depend on the clinical significance of the dental condition, and the clinical significance of a dental condition may be established by a dental practitioner or by any other dental health expert. For example, the clinical significance of a dental condition may be chosen based on the rate at which the stage of the dental condition advances or based on a possible insurgence of other conditions due to the presence of that dental condition. For example, the caries may be weighted with a weight $w_1$=3, the cracks may be weighted with a weight of $w_2$=1, the wear may be weighted with a weight of $w_3$=2, the recession may be weighted with a weight of $w_4$=2 and the plaque may be weighted with a weight of $w_5$=1. Thus, also with reference to the example above, the aggregated severity level $(w_1 \times 3 + w_2 \times 0 + w_3 \times 9 + w_4 \times 3 + w_5 \times 1) = 34$ is determined for the data point. It will be appreciated by those skilled in the art that the sum of the severity level of each dental condition has the advantage of reducing the processing time of the data points. On the other hand, the weighted sum has the advantage of providing an accurate and realistic indication of an overall health condition of a data point, as it accounts for realistic clinical features of each dental condition.

In step 104, an epicenter of the one or more aggregated severity levels of each inspection site is determined by determining a maximum aggregated severity level of the inspection site. The epicenter of each inspection site may comprise more than one data point if more data points of the inspection site are determined to have the same maximum aggregated severity level. For example, an inspection site may contain 6 data points $P_1$, $P_2$, $P_3$, $P_4$, $P_5$, $P_6$ for which the aggregated severity levels 10, 15, 15, 15, 5, 4 have been determined, respectively. In this example, the maximum severity level of the inspection site is determined to be 15, which is associated with the data points $P_2$, $P_3$, $P_4$. Therefore, the epicenter of the inspection site comprises the data point $P_2$, $P_3$, $P_4$ and is associated with the aggregated severity level 15.

In step number 105, the one or more data points of the maximum aggregated severity level determined for each inspection site are associated with a location of the epicenter. Thus, one or more locations of the dental site for which the highest aggregated severity level has been locally determined are accurately determined. These locations may be reported to the dental practitioner performing the scanning, e.g. as a graphical representation displayed on a rendering of the 3D model of the dental site on a display of the computer system on which the method is performed. In this way, the dental practitioner may be provided with a clear indication of the overall health status of the patient's dental site at once, and with an indication of the one or more regions of the dental site which necessitate a careful inspection, either to treat existing dental conditions and/or to prevent the development of other conditions.

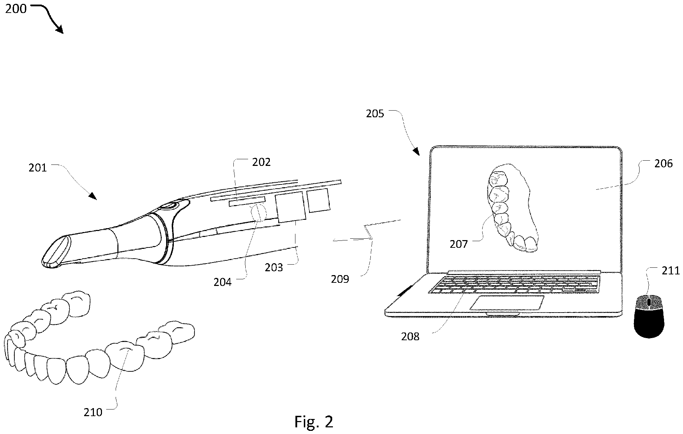

Figure 2 illustrates an example of intraoral scanning system 200 according to the present disclosure. The intraoral scanning system 200 comprises an intraoral scanner 201 and a computer system 205. The intraoral scanner 201 may be configured to acquire intra-oral scan data relative to a dental site 210 of a patient, for example during a live scanning session which may be carried out by a dental practitioner when the patient visits a dental studio. As also mentioned above, the intraoral scanning device 201 may employ any scanning principle such as triangulation-based scanning, confocal scanning, focus scanning, ultrasound scanning, stereo vision, structure from motion, optical coherent tomography OCT, or any other scanning principle suitable for acquiring intraoral scan data. In one example, the intraoral scanner 201 may be configured to acquire surface information of the dental site 210 by operated by projecting a pattern and translating a focus plane along an optical axis of the intraoral scanner 201, and capturing a plurality of two-dimensional (2D) images at different focus plane positions such that each series of captured 2D images corresponding to each focus plane forms a stack of 2D images. The stack of 2D images is also referred to herein as sub-scan. During the scanning, a number of sub-scans is acquired for a number of given angle view of the dental site 210, i.e. for a given arrangement of the intraoral scanner 201 relative to the dental site 210. The intraoral scanner 201 is generally moved and angled relative to the dental site 210, such that least sets of sub-scans overlap at least partially, in order to generate a reconstruction of the 3D model of the dental site 210 by stitching overlapping sub-scans together in real-time. The stitching process, also known as registration and fusion, may be implemented by means of an Iterative Closest Point (ICP) algorithm. As the generation of the reconstruction of the 3D model progresses, the progress of the reconstruction of the 3D model may be displayed on a display 206 of the computer system 205. In another example, intraoral scanner 201 is a triangulation scanner, where a time varying pattern is projected onto the dental object and a sequence of images of the different pattern configurations are acquired by one or more cameras located at an angle relative to the projector unit.

Color texture of the dental site 210 may be acquired by illuminating the dental site 210 using different monochromatic colors such as individual red, green and blue colors or by illuminating the object using multichromatic light such as white light. A 2D image may be acquired during a flash of white light.

More generally, the intraoral scanner 201 comprises one or more light projectors 204 configured to generate an illumination pattern to be projected on at least a part of the dental site 210 during a scanning session. The light projector(s) preferably comprises a light source, a mask having a spatial pattern, and one or more lenses such as collimation lenses or projection lenses. The light source may be configured to generate light of a single wavelength or a combination of wavelengths (mono- or polychromatic). The combination of wavelengths may be produced by using a light source configured to produce light (such as white light) comprising different wavelengths. Alternatively, the light projectors may comprise multiple light sources such as Light Emitting Diodes (LEDs) individually producing light of different wavelengths (such as red, green and blue) that may be combined to form light comprising the different wavelengths. Thus, the light produced by the light source may be defined by a wavelength defining a specific color, or a range of different wavelengths defining a combination of colors such as white light. In an embodiment, the scanning device comprises a light source configured for exciting fluorescent material of the teeth to obtain fluorescence data from the dental object. Such a light source may be configured to produce a narrow range of wavelengths. In another embodiment, the light from the light source is infrared (IR) light, which is capable of penetrating dental tissue. The light projector(s) may be Digital Light Processing (DLP) projectors using a micro mirror array to generate a time varying pattern, or a diffractive optical element (DOF), or back-lit mask projectors, wherein the light source is placed behind a mask having a spatial pattern, whereby the light projected on the surface of at least a part of the dental site 210 is patterned. The back-lit mask projector may comprise a collimation lens for collimating the light source, said collimation lens being placed between the light source and the mask. The mask may have a checkboard pattern, such that the generated illumination pattern is a checkboard pattern. Alternatively, the mask may feature other patterns such as lines or dots, etc.

The intraoral scanner 201 may further comprise optical components for directing the light from the light source to the surface of the dental object. The specific arrangement of the optical components depends on whether the intraoral scanner 201 is a focus scanning device, a scanner using triangulation, or any other type of intraoral scanning device.

The light reflected from the dental site 210 in response to the illumination of the dental site is directed, using the optical components of the intraoral scanner 201, towards the image sensor(s) 202. The image sensor(s) 202 are configured to generate a plurality of images based on the incoming light received from the illuminated dental object. The image sensor(s) 202 may be a high-speed image sensor such as an image sensor configured for acquiring images with exposure of less than 1/1000 second or frame rates in excess of 250 frames pr. second (fps). As an example, the image sensor may be a rolling shutter (CCD) or global shutter sensor (CMOS). The image sensor(s) 202 may be a monochrome sensor including a color filter array such as a Bayer filter and/or additional filters that may be configured to substantially remove one or more color components from the reflected light and retain only the other non-removed components prior to conversion of the reflected light into an electrical signal. For example, such additional filters may be used to remove a certain part of a white light spectrum, such as a blue component, and retain only red and green components from a signal generated in response to exciting fluorescent material of the teeth.

The intraoral scanning system 200 may further comprise one or more processors 203 configured to generate scan data (such as intraoral scan data) by processing the data acquired by the intraoral scanner 201, e.g. by processing the 2D images acquired by the intraoral scanner 201. The one or more processors 203 may be a part of the intraoral scanner 201. For example, the processor(s) 203 may comprise a Field-programmable gate array (FPGA) and/or an Advanced RISC Machine (ARM) processor located in the intraoral scanner 201. The scan data may comprise any of: 2D images, 3D point cloud, depth data, texture data, intensity data, color data, and/or combinations thereof. As an example, the scan data may comprise one or more point clouds, wherein each point cloud comprises a set of 3D points describing at least a part of the 3D dental site 210. As another example, the scan data may comprise images, each image comprising image data e.g. described by image coordinates and a timestamp (x,y,t), wherein depth information can be inferred from the timestamp. The intraoral scanner 201 may be configured for acquiring a number of sub-scans (i.e. a stack of raw 2D images, wherein raw means that the 2D images are not processed) by means of the image sensor(s) 202. In this case, the sub-scans are provided as input to the processor(s) 203. The processing of the raw 2D images may comprise the step of determining which part of each of the 2D images are in focus in order to generate/deduce depth information from the images. Said depth information may be used to generate 3D point clouds comprising a set of 3D points in space described by a set of Cartesian coordinates (x,y,z). The 3D point clouds may be generated by the processor(s) 203 or by any other processing unit. Each 2D/3D point may further comprise a timestamp indicating when the 2D/3D point was recorded, i.e. from which image in the stack of 2D images the point originates. The timestamp is correlated with the z-coordinate of the 3D points, i.e. the z-coordinate may be inferred from the timestamp. Accordingly, the output of the processor(s) 203 is the scan data, and the scan data may comprise image data and/or depth data, e.g. described by image coordinates and a timestamp (x, y, t) or alternatively described as (x, y, z).

The intraoral scanner 201 may be configured to transmit, either through wired connection or wireless connection 209, scan data and other types of data to the computer system 205. Examples of data include any of 3D information, texture information such as IR images, fluorescence images, reflectance color images, x-ray images, and/or combinations thereof. The computer system 205 may comprise a display 206 to display a rendering 207 of the 3D model of the dental site 210 generated during the scanning session, and/or a rendering 207 of the 3D model of the dental site 210 generated in a scanning session of the dental site 210 performed at a previous time.

The system 200 may further be configured to display a live view of the intraoral scanner 201. The scanner system 200 may be further configured to detect one or more input signal(s). As an example, the input signal may be detected through one or more sensors (not shown here) provided in the intraoral scanner 201. In this example, the intraoral scanner may be further configured to transmit the input signal to the computer system 205. In another example, the input signal may be further or alternatively detected through one or more external devices connected to the computer system 205, such as a keyboard 208 and/or a mouse 211 or any other input device.

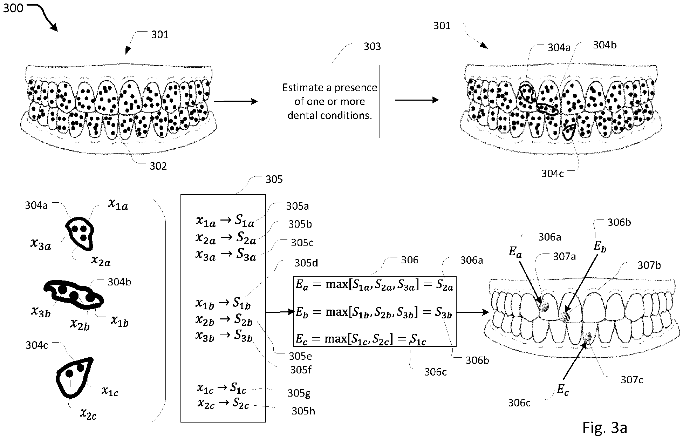

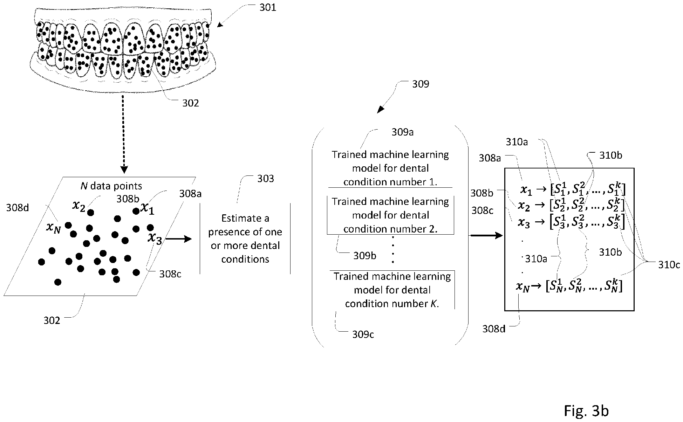

Figures 3a and 3b illustrate example reference diagrams 300 for describing an embodiment of a workflow of the computer-implemented method disclosed herein. Referring to Figure 3a, a 3D model 301 of a dental site is obtained. Said 3D model may be generated during a scanning session of the dental site of a patient performed during a clinical visit of the patient, or it may have been generated in a scanning session performed at a previous time and stored, e.g. in a memory of a computer system on which the method disclosed herein is performed, such as the computer system indicated as reference number 205 in Figure 2.

The obtained 3D model comprises a plurality of data points 302. In one example, the plurality of data points 302 may be a point cloud/a plurality of point clouds in 3D space, wherein each point cloud comprises a set of points in 3D space described by a set of Cartesian coordinates (x, y, z). In another example, the plurality of data points 302 may comprise a plurality of facets comprised in a mesh describing the surface of the dental site in 3D space. Each facet may be a triangle in the case of a triangulated mesh, or any other polygon in the case of a polygonal mesh. In yet another example, the plurality of data points 302 may comprise a plurality of vertices comprised in a mesh describing the surface of the dental site in 3D space. In general, the plurality of data points may comprise any other data which is suitable to describe the surface of the dental object in 3D space.

Once the 3D model 301 has been obtained, it is processed in step 303 to estimate a presence of one or more dental conditions of the dental site. The one or more dental conditions may comprise one or more of caries, cracks, recession, wear, plaque, and any other dental condition which may be of interest to the dental practitioner to assess the health status of the dental site. The processing 303 may output a number of inspection sites, wherein each inspection site comprises one or more of the plurality of data points 302 for which at least one dental condition has been estimated in 303. The processing 303 may comprise processing each of the plurality of data points, and therefore the output of 303 comprises an output for each of the plurality of data points 302. For example, with reference to Figure 3a, the processing 303 may define three inspection sites of the 3D model 301: 304a comprising three data points ($x_{1a}$, $x_{2a}$, $x_{3a}$), 304b comprising three data points ($x_{2a}$, $x_{2b}$, $x_{2c}$), and 304c comprising two data points ($x_{3a}$, $x_{3b}$). In general, the inspection sites defined by the output of 303 may be intended as regions of the 3D model 301 which are affected by at least one dental condition. The example given in Figure 3a illustrates two inspection sites comprising three data points and one inspection site comprising two data points, but it will be appreciated by those skilled in the art that an inspection site may comprise more or less than three/two data points.

In step 305 an aggregated severity level is determined for each data point comprised in each inspection site, whereby one or more aggregated severity levels are determined for each inspection site. The aggregated severity level of a data point may also be referred to herein as "heat score" of the data point. The heat score of a data point quantifies the overall health status of that data point, and it therefore indicates the aggregated/overall severity of the one or more dental conditions estimated for the data point. For example, if for the data point $x_{1a}$ the presence of caries, cracks and plaque is estimated by the processing 303, the heat score $S_{1a}$ determined for $x_{1a}$ indicates the aggregated severity determined for caries, cracks and plaque for $x_{1a}$. With reference to Figure 3a, the heat scores 305a, 305b, 305c are determined for the inspection site 304a, the heat scores 305d, 305e, 305f are determined for the inspection site 304b and the heat scores 305g, 305h are determined for the inspection site 304c, namely the heat scores:

$$(x_{1a}, x_{2a}, x_{3a}) \rightarrow (S_{1a}, S_{2a}, S_{3a})$$

$$(x_{1b}, x_{2b}, x_{3b}) \rightarrow (S_{1b}, S_{2b}, S_{3b})$$

$$(x_{1c}, x_{2c}) \rightarrow (S_{1c}, S_{2c})$$

are determined in 305 for each data point comprised in the inspection site 304a, 304b and 304c, respectively.

In step 306, an epicenter of the one or more dental conditions is determined for each inspection site by determining a maximum heat score of each inspection site, namely:

$$E_a = \max[S_{1a}, S_{2a}, S_{3a}] = S_{2a}$$

$$E_b = \max[S_{1b}, S_{2b}, S_{3b}] = S_{3b}$$

$$E_c = \max[S_{1c}, S_{2c}] = S_{1c}$$

wherein $E_a$ is the epicenter 306a determined for the inspection site 304a, $E_b$ is the epicenter 306b determined for the inspection site 304b, and $E_c$ is the epicenter 306c determined for the inspection site 304c. In the example shown in Figure 3a, the maximum heat score of the inspection site 304a is determined to be $S_{2a}$, i.e. the epicenter 306a is $E_a = S_{2a}$, and the maximum heat score of the inspection site 304b is determined to be $S_{3b}$, i.e. the epicenter 306b is $E_b = S_{3b}$, and the maximum heat score of the inspection site 304c is determined to be $S_{1c}$, i.e. the epicenter 306c is $E_c = S_{1c}$. It will be appreciated by those skilled in the art that two or more data points of an inspection site may be assigned with the same heat score and that said same heat score may be determined to be the maximum heat score of the inspection site. In this case, the epicenter of the inspection site comprises the two or more data points with the same maximum heat score.

Once the epicenter of each inspection site is determined, the one or more data points of the maximum heat score of each inspection site are associated with a location of the epicenter . With reference to Figure 3a, the data point $x_{2a}$ may be associated with the location 307a of the epicenter $E_a$ 306a, the data point $x_{3b}$ may be associated with the location 307b of the epicenter $E_b$ 306b, and the data point $x_{1c}$ may be associated with the location 307c of the epicenter $E_c$ 306c. Therefore, the regions of the dental site for which the overall health status is determined to be locally the most severe, i.e. the regions of the dental site which necessitate the highest attention, are accurately determined. The locations of the epicenters of the one or more inspection sites are the regions of the dental site which may benefit from a careful examination by the dental practitioner, and potentially demand a treatment plan. Thus, the dental practitioner may use the location of the epicenter of each inspection site as a guide to perform the manual examination of the dental site of the patient. For example, the examination may be started from the location of the epicenter of each inspection site, and it may proceed by checking the health condition of the region around said epicenter. Such a guide significantly improves and fasten the diagnostic efficiency, allowing the dental practitioner to quickly identify and inspect the problematic locations of the dental site, potentially preventing the development of additional dental conditions in the location of the epicenter.

Even though in the example illustrated in Figure 3a the epicenter is determined for each inspection site of the 3D model, in an alternative embodiment (not illustrated here) the method disclosed herein may comprise determining only one epicenter of the dental conditions, namely a global epicenter of the dental conditions, by determining a global maximum heat score among the one or more heat scores determined for all of the inspection sites of the 3D model. In this case, the global epicenter may be intended as the region of the 3D model for which the health status is, globally, the worst and which therefore necessitates the highest attention by the dental practitioner. In other words, the method described in the example illustrated in Figure 3a may comprise determining at least one epicenter of the dental conditions in the 3D model of the dental site, and said epicenter may be intended as a local and/or as a global epicenter.

Figure 3b is a reference diagram of an embodiment of the processing for estimating a presence of one or more dental conditions in a dental site as the one illustrated as reference number 303 in Figure 3a. Estimating the presence of the one or more dental condition in the dental site comprises processing each of the plurality of data points 302 comprised in the 3D model 301 of the dental site using a trained machine learning model for each dental condition 309. Accordingly, if the 3D model 301 comprises $N$ data points 302 $x_1$ (308$a$), $x2$ (308$b$), $x_3$(308$c$), ... , $x_N$(308$d$), each of these data points is input in a trained machine learning model for each dental condition.

Referring to Figure 3b, the processing 303 comprises estimating a presence of a number of $K$ dental conditions. Accordingly, each of the $N$ data points 302 are inputted in a trained machine learning model 309a for the dental condition number 1, in a trained machine learning model 309b for dental condition number 2, and so forth, up to the trained machine learning model 309c for dental condition number $K$. For each data point, each of the trained machine learning models for a specific dental condition outputs a severity level for that dental condition. In particular, the trained machine learning model 309a outputs for each data point in 302 a severity level 310a for the dental condition number 1, the trained machine learning model 309b outputs for each data point in 302 a severity level 310b for dental condition number 2, and so forth, up to the trained machine learning model 309c which outputs for each data point in 302 a severity level 310c for dental condition number $K$. Therefore, referring to Figure 3a the output of the trained machine learning models 309, i.e. the output of the processing 303, is:

$$x_1 \rightarrow [S_1^1, S_1^2, ..., S_1^k]$$
$$x_2 \rightarrow [S_2^1, S_2^2, ..., S_2^k]$$
$$x_3 \rightarrow [S_3^1, S_3^2, ..., S_3^k]$$
$$.$$
$$.$$
$$.$$
$$x_N \rightarrow [S_N^1, S_N^2, ..., S_N^k]$$

wherein $S_1^1, S_2^1, ..., S_N^1$ are the severity levels 310a of dental condition 1 determined by the machine learning model 309a for the data point $x_1$ (308$a$), $x_2$ (308$b$), $x_3$(308$c$), ... , $x_N$ (308$d$), respectively, $S_1^2, S_2^2, ..., S_N^2$ are the severity levels 310b of dental condition 2 determined by the machine learning model 309b for the data point $x_1$ (308$a$), $x_2$ (308$b$), $x_3$ (308$c$), ..., $x_N$(308$d$), respectively, and so forth, up to $S_1^k, S_2^k, ..., S_N^k$ which are the severity levels 310c determined by the machine learning model 309c of dental condition $K$ for the data point $x_1$ (308$a$), $x_2$ (308$b$), $x_3$(308$c$), ..., $x_N$ (308$d$), respectively. The example illustrated in Figure 3b refers to a trained machine learning model to detect the presence of the one or more dental conditions, however the presence of each dental condition in the dental site may be estimated by using other methods, e.g. mathematical and/or statistical methods using color information comprised in the scan data used to generate the 3D model of the dental site.

Referring again to Figure 3a, a severity level for each of the $K$ dental conditions may be determined for each data point in the defined inspection sites, i.e. the inspection sites 304a, 304b and 304c, wherein the severity level of each of the $K$ dental conditions is determined accordingly to the workflow of the processing 303 illustrated in Figure 3b. For example, for the data point $x_{1a}$ the severity levels $S_{1a}^1, S_{1a}^2, ..., S_{1a}^k$ may be determined in 303 for the $K$ dental conditions. Accordingly, the aggregated severity level $S_{1a}$ 305a determined for the data point $x_{1a}$ is determined by aggregating the severity levels $S_{1a}^1, S_{1a}^2, ..., S_{1a}^k$. Similarly, for the data point $x_{1b}$ the severity levels $S_{1b}^1, S_{1b}^2, ..., S_{1b}^k$ may be determined in 303 for the $K$ dental conditions. Accordingly, the aggregated severity level $S_{1b}$ 305d determined for the data point $x_{1b}$ is determined by aggregating the severity levels $S_{1b}^1, S_{1b}^2, ..., S_{1b}^k$. Similar discussion holds for the aggregated severity levels 305c, 305d, 305e, 305f, 305g, 305h determined for the data points $x_{2a}, x_{3a}, x_{2b}, x_{3b}, x_{1c}, x_{2c}$, respectively. Aggregating the severity level of each of the $K$ dental conditions may comprise summing the severity level determined for each of the $K$ dental conditions, or it may comprise determining a weighted sum of the severity level determined for each of the $K$ dental conditions, wherein a weight of each dental condition may be determined based on a clinical significance of that dental conditions or based on clinical features of that dental condition.

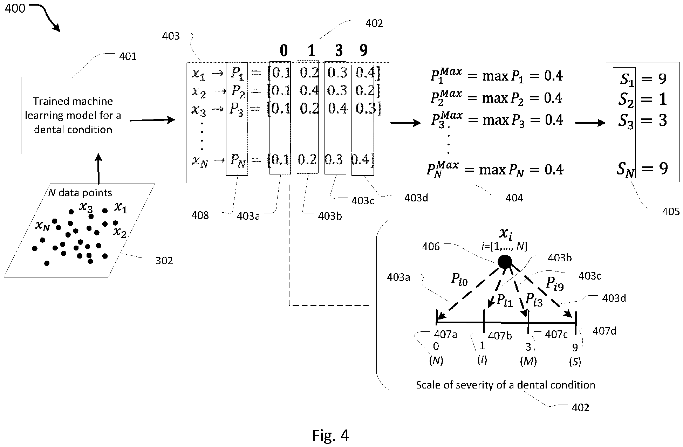

Figure 4 illustrates an example reference diagram 400 of an embodiment of a trained machined learning model for a dental condition according to the present disclosure. The trained machine learning model for the dental condition 401 receives as input a number $N$ of data points 302 ($x_1, x_2, ..., x_N$). The $N$ data points 302 may be $N$ points of a 3D point cloud, or the data points may be $N$ facets of a triangulated mesh, or the data points may be $N$ vertices of a triangulated mesh, or the data points may be any other data suitable to describe a surface of the dental site in 3D space. For each data point $x_i$ 406,

the trained machine learning model 401 outputs a probability $P_i$ 408, where here the index $i = [1, ..., N]$. Said probability $P_i$ 408 may be a vector, and each element of the vector may be a probability that the dental condition is present in that data point with a specific severity level, wherein the latter is a number on a predefined scale of severity 402 of the dental condition. Therefore, the probability $P_i$ 408 comprises as many elements as the number of severity levels on the predefined scale of severity of the dental condition 402. In general, the scale of severity of a specific dental condition comprises numbers, and each of said numbers quantifies the severity of the specific dental condition. For example, referring to Figure 4, the scale of severity of the dental condition may comprises the numbers 0 (407a), 1 (407b), 3 (407c), 9 (407d), corresponding to an absence of the dental condition, an initial dental condition, a moderate dental condition and a severe dental condition, respectively. Thus, the probability $P_i$ 408 which is output for each data point $x_i$ 406 by the trained machine learning model 401 may be a vector $P_i = [P_{i0}\ P_{i1}\ P_{i3}\ P_{i9}]$, wherein $P_{i0}$ is the probability 403a that the dental condition is present in the data point $x_i$ with the severity level 0 407a, $P_{i1}$ is the probability 403b that the dental condition is present in the data point $x_i$ with the severity level 1 407b, $P_{i3}$ is the probability 403c that the dental condition is present in the data point $x_i$ with the severity level 3 407c, and $P_{i9}$ is the probability 403d that the dental condition is present in the data point $x_i$ with the severity level 9 407d. Here, the data point $x_i$ 406 is any of the $N$ data points 302, i.e. $i = [1,..,N]$.

Accordingly, with reference to Figure 4, the output of the trained machine learning model 401 for the data point $x_1$ is a vector of probabilities 408 $P_1 = [0.1\ 0.2\ 0.3\ 0.4]$, wherein 0.1 is the probability 403a for $x_1$ to be associated with the severity level 0 407a of the dental condition, 0.2 is the probability 403b for $x_1$ to be associated with the severity level 1 407b of the dental condition, 0.3 is the probability 403c for $x_1$ to be associated with the severity level 3 407c of the dental condition, and 0.4 is the probability 403d for $x_1$ to be associated with the severity level 9 407d of the dental condition. Similarly, the output of the trained machine learning model 401 for the data point $x_2$ is a vector of probabilities 408 $P_2 = [0.1\ 0.4\ 0.3\ 0.2]$, wherein 0.1 is the probability 403a for the data point $x_2$ to be associated with the severity level 0 407a of the dental condition, 0.4 is the probability 403b for $x_2$ to be associated with the severity level 1 407b of the dental condition, 0.3 is the probability 403c for the data point $x_2$ to be associated with the severity level 3 407c of the dental condition, and 0.2 is the probability 403d for the data point $x_2$ to be associated with the severity level 9 407d of the dental condition. Similar arguments are held for all the remaining $N$-2 data points in 302. Therefore, the output of the trained machine learning model 401 for the input $N$ data points is the set of vectors 403:

$$x_1 \rightarrow P_1 = [0.1\ 0.2\ 0.3\ 0.4]$$
$$x_2 \rightarrow P_2 = [0.1\ 0.4\ 0.3\ 0.2]$$
$$x_3 \rightarrow P_3 = [0.1\ 0.2\ 0.4\ 0.3]$$
$$.$$
$$.$$
$$.$$
$$x_N \rightarrow P_N = [0.1\ 0.2\ 0.3\ 0.4]$$

The maximum probability in the vector of probabilities 408 estimated for each data point $x_i$ 406 is then determined in 404 for each data point 406, namely:

$$P_1^{Max} = \max P_1 = \max[0.1\ 0.2\ 0.3\ 0.4] = 0.4$$

$$P_2^{Max} = \max P_2 = \max[0.1\ 0.4\ 0.3\ 0.2] = 0.4$$

$$P_3^{Max} = \max P_3 = \max[0.1\ 0.2\ 0.4\ 0.3] = 0.4$$

$$.$$
$$.$$
$$.$$

$$P_2^{Max} = \max P_2 = \max[0.1\ 0.4\ 0.3\ 0.2] = 0.4$$

Accordingly, each data point $x_i$ 406 is assigned with a severity level 405 of the dental condition, wherein for each data point the severity level 405 is the severity level for which the maximum probability 404 is determined for that data point.

For example, the maximum probability 404 $P_1^{Max} = 0.4$ determined for $x_1$ is the probability that the dental condition is present in the data point $x_1$ with a severity level 9. Accordingly, the severity level 405 $S_1 = 9$ is determined for the data point

$x_1$. Similarly, the maximum probability 404 $P_2^{Max} = 0.4$ determined for $x_2$ is the probability that the dental condition is present in the data point $x_2$ with the severity level 1. Accordingly, the severity level 405 $S_2 = 1$ is determined for the data point $x_2$ in 305. Similar arguments hold for all the $N$ data points in 302, such that the severity levels 405:

$$S_1 = 9$$
$$S_2 = 1$$
$$S_3 = 3$$
$$.$$
$$.$$
$$.$$
$$S_N = 9$$

are determined for the $N$ data points 302 input in the trained machine learning model 401.

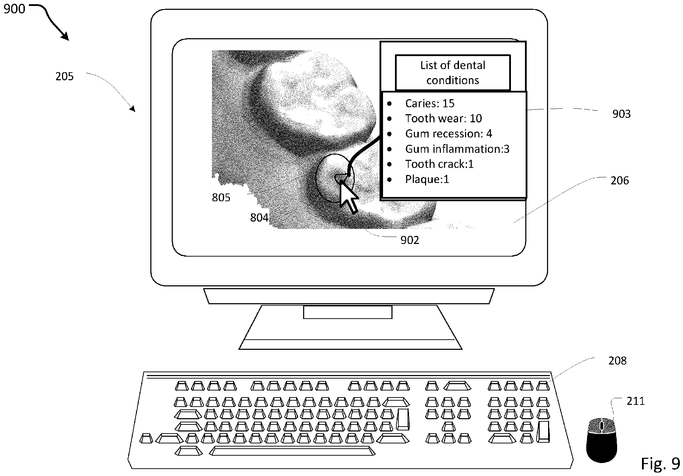

The numbers 0, 1, 3, 9 are used in the example illustrated in Figure 9 just for illustrative purposes of an embodiment of the trained machine learning model according to the present disclosure. In practice, the numbers defining the scale of severity of the dental condition may be chosen by a dental practitioner, or by any other dental expert, based on a clinical significance of the specific dental condition and/or based on the clinical features of that condition. For example, the scale of severity for each dental condition may comprise four numbers corresponding to an absence of the dental condition, an initial stage of development of the dental condition, a moderate stage of development of the dental condition and a severe stage of development of the dental condition, respectively. In this example, the dental expert may infer that caries are more clinically significant than plaque, e.g. because caries may cause the development of other dental conditions. Thus, the dental expert may decide to define a scale of severity for caries containing larger numbers than the scale of severity for plaque, e.g. the scale of severity for caries may be chosen to comprise the numbers (0, 4, 9, 16) and the scale of severity for plaque may be chosen to comprise the numbers (0, 1, 2, 3), corresponding to absent, initial, moderate and severe levels, respectively. Accordingly, if a presence of initial caries is determined for a first data point and a presence of a severe plaque is determined for a second data point, the first data point will be assigned with the severity level 4 and the second data point with the severity level 3, such that a higher clinical significance will be assigned to the first data point than to the second data point. The scale of severity defined by the dental practitioner may be input to one or more processors, e.g. the processor(s) of a computer system performing the method described herein. The method described herein may further or alternatively comprise a predefined scale of severity for each dental condition.

In another example, the numbers defining the scale of severity of a dental condition may be fixed numbers raised to a variable exponent. Said exponent may be varied, e.g. by the dental practitioner or the dental expert, depending on the clinical significance of a specific dental condition, and/or depending on a desired gap between the severity levels of a dental condition. For example, the predefined numbers may be 0, 1, 2, 3, corresponding to an absent dental condition, an initial dental condition, a moderate dental condition, and a severe dental condition, respectively, and each of the predefined number may be raised by an exponent e, namely the severity scale may be defined as:

$$0^e \ \ 1^e \ \ 2^e \ \ 3^e$$

In one example, the dental practitioner may want to have an indication of a specific dental condition at all its stages of development, i.e. they may want to minimize the gap between the absent, initial, moderate and severe levels. Accordingly, they may choose the exponent to be $e = 1$. In another example, the dental practitioner may be interested only in the severe stage of a dental condition, i.e. they want to increase the gap between the absent, initial, moderate and severe levels of the scale of severity of the dental condition. Accordingly, they may choose the exponent to be $e = 3$, such that the severe condition will correspond to a severity of 27, whereas a moderate condition will be associated with a severity of 8, i.e. the severe condition will be quantified as much more relevant than the moderate one. In yet another example, the exponent e may be chosen to be different for different dental conditions, for example based on a clinical significance of each dental condition. For example, the dental practitioner may choose to assign a higher clinical significance to caries than to plaque, and accordingly fix the exponent $e = 3$ for caries and $e = 1$ for plaque. Thus, if a first data point is estimated to have severe caries and a second data point is estimated to have severe plaque, the former will have a larger severity than the latter, i.e. the data points with severe caries will be given more significance for inferring the health status of the dental site than the data points with severe plaque. It will be appreciated by those skilled in the art that the above-mentioned examples are intended to be illustrative, and that the exponent may be chosen in an appropriate way depending on the clinical features of the inspected dental condition. Alternatively, the exponent *e* may be a set feature in the method described herein, or it may be received as an input from a user of a system, e.g. a computer system, configured to perform the steps of the method disclosed herein.

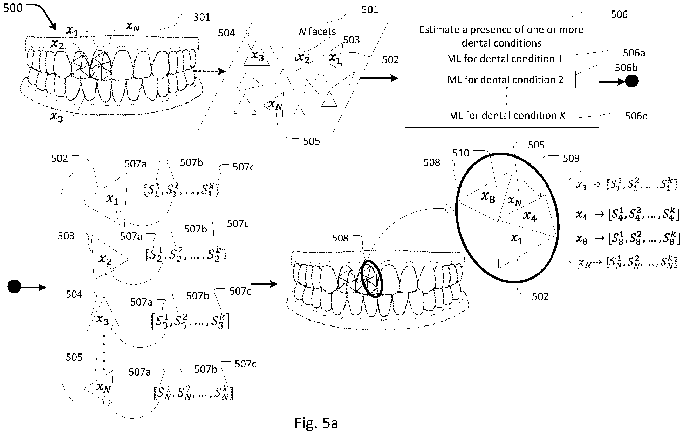

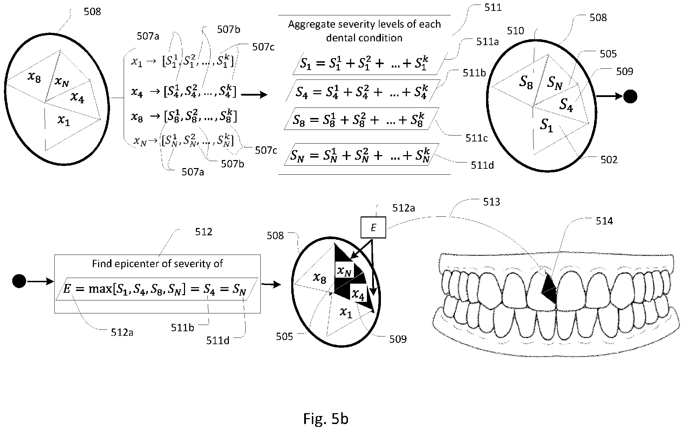

Figures 5a and 5b illustrate example reference diagrams 500 for describing an embodiment of a workflow of the computer-implemented method disclosed herein. As a first step of 500, a 3D model 301 of the dental site is obtained. The 3D model 301 comprises a triangulated mesh, wherein the triangulated mesh comprises *N* facets 501, and in particular it comprises a facet $x_1$ (502), a facet $x_2$ (503), a facet $x_3$ (504), and so forth, up to a facet $x_N$ (505). The *N* facets 501 collectively describe the surface of the dental site in 3D space, and therefore they comprise information about at least the 3D geometry of the dental site. In step 506, the presence of one or more dental conditions in the 3D model 301 of the dental site is estimated. In particular, the presence of the dental conditions 1,...,*K* is estimated in step 506 by inputting the *N* facets 501 in a trained machine learning model 506a for dental condition number 1, and in a trained machine learning model 506b for dental condition number 2, and so forth, up to a trained machine learning model 506c for dental condition number *K*.

The trained machine learning model for each dental condition outputs for each data point a severity level of that dental condition. For example, the trained machine learning model 506a for dental condition number 1 outputs the severity level $S_1^1$ 507a of the dental condition 1 for the facet 502 $x_1$, the severity level $S_2^1$ 507a of the dental condition 1 for the facet 503 $x_2$, the severity level $S_3^1$ 507a of the dental condition 1 for the facet 504 $x_3$, and so forth, up to the severity level $S_N^1$ 507a of the dental condition 1 for the facet 505 $x_N$. Similarly, the trained machine learning model 506b for dental condition number 2 outputs the severity level $S_1^2$ 507b of the dental condition 2 for the facet 502 $x_1$, and the severity level $S_2^2$ 507b of dental condition 2 for the facet 503 $x_2$, and the severity level $S_3^2$ 507b of dental condition 2 for the facet 504 $x_3$, and so forth, up to the severity level SN 507b of dental condition 2 for the facet 505 $x_N$. Similar arguments hold for the trained machine learning model for condition 3, the trained machine learning model for condition 4, and so forth, up to the trained machine learning model 506c for dental condition *K*, which outputs the severity level $S_1^K$ 507c of the dental condition *K* for the facet 502 $x_1$, and the severity level $S_2^K$ 507c of dental condition K for the facet 503 $x_2$, and the severity level $S_3^K$ 507c of dental condition *K* for the facet 504 $x_3$, and so forth, up to the severity level $S_N^K$ 507c of dental condition *K* for the facet 505 $x_N$. In other words, the output of 506 is:

$$x_1 \rightarrow [S_1^1, S_1^2, ..., S_1^K]$$
$$x_2 \rightarrow [S_2^1, S_2^2, ..., S_2^K]$$
$$x_3 \rightarrow [S_3^1, S_3^2, ..., S_3^K]$$
$$.$$
$$.$$
$$.$$
$$x_N \rightarrow [S_N^1, S_N^2, ..., S_N^K]$$

The above-mentioned severity levels are numbers on a scale of severity of each dental condition, and said scale of severity comprises a minimum number which indicates an absence of the dental condition. The minimum number may be, for example, zero, such that a facet for which all the severity levels $S_i^j = 0$, where $i \in [1, N]$ and $j = [1, ..., K]$, is a facet for which no dental condition is present. If the minimum number on the scale of severity is different from zero $S_{MIN} \neq 0$, then a facet for which all the severity levels $S_i^j = S_{MIN}$, with $i \in [1, N]$ and $j = [1, ..., K]$, is a facet for which no dental condition is present.

The one or more facets among the *N* facets 501 for which the presence of at least one dental condition is estimated in 506 define one or more inspection sites of the 3D model 301. For example, with reference to Figure 5a, an inspection site 508 of the 3D model 301 may comprise four facets $x_1$(502), $x_4$(509), $x_N$(505), $x_8$(510) for which the presence of at least one dental condition was estimated in 506. Thus, at least one of the severity levels $[S_1^1, S_1^2, ..., S_1^K]$ determined for the facet 502 is different than the minimum severity level of the scale of severity of a dental condition, and at least one of the severity levels $[S_4^1, S_4^2, ..., S_4^K]$ determined for the facet 509 is different than the minimum severity level of the scale of severity of a dental condition, and at least one of the severity levels $[S_8^1, S_8^2, ..., S_8^K]$ determined for the facet 505 is different than the minimum severity level of the scale of severity of a dental condition, and at least one of the severity levels $[S_N^1, S_N^2, ..., S_N^K]$ determined for the facet 510 is different than the minimum severity level of the scale of severity of a dental condition.

Figure 5b illustrates an example reference diagram of a method for determining an epicenter of the one or more dental conditions. First, for each facet in each inspection site, an aggregated severity level/heat score of the $K$ dental conditions is determined in 511. In particular, the heat score is determined for each of the facets $x_1(502)$, $x_4(509)$, $x_N(505)$, $x_8(510)$ which are comprised in the inspection site 508 defined accordingly to Figure 5a. With reference to the example illustrated in Figure 5b, the heat score of each facet in the inspection site 508 is determined by summing the severity levels of each dental condition 1,...,$K$ determined for that facet. Therefore, the heat score 511a $S_1 = S_1^1 + S_1^2 + \cdots + S_1^K$ is determined for the facet $x_1$, the heat score 511b $S_4 = S_4^1 + S_4^2 + \cdots + S_4^K$ is determined for the facet $x_4$, the heat score 511c $S_8 = S_8^1 + S_8^2 + \cdots + S_8^K$ is determined for the facet $x_8$ and the heat score 511d $S_N = S_N^1 + S_N^2 + \cdots + S_N^K$ is determined for the facet $x_N$. In other words, four heat scores $S_1$ (511a), $S_4$(511b), $S_8$(511c), $S_N$(511d) are determined in 511 for the inspection site 508 by aggregating, for each facet comprised in the inspection site 508, the severity level $S_i^1 (507a), S_i^2(507b), ..., S_i^k(507c)$, where $i = 1,4,8,N$, i.e. the severity level determined for the dental conditions 1, 2,...,$K$ for the facets 502, 509, 505 and 510 of the inspection site 508.

In another example (not shown here), the heat score for each facet in the inspection site 508 may be determined by determining a weighted sum of the severity levels of each dental condition determined for that facet. In this example, the weights $w_1$, ..., $w_k$ may be determined for the dental condition 1,..., $K$, respectively, such that the heat score

$S_1 = w_1 \times S_1^1 + w_2 \times S_1^2 + \cdots + w_K \times S_1^K$ is determined for the facet $x_1$, the heat score

$S_4 = w_1 \times S_4^1 + w_2 \times S_4^2 + \cdots + w_K \times S_4^K$ is determined for the facet $x_4$, the heat score

$S_8 = w_1 \times S_8^1 + w_2 \times S_8^2 + \cdots + w_K \times S_8^K$ is determined for the facet $x_8$ and the heat score

$S_N = w_1 \times S_N^1 + w_2 \times S_N^2 + \cdots + w_K \times S_N^K$ is determined for the facet $x_N$. The weights of each dental condition may be defined by the dental practitioner, or by any dental expert performing the examination of the dental site, based on the clinical significance of each dental condition, such that the higher the clinical significance attributed to the dental condition, the larger the weight. Further or alternatively, the weights assigned to each dental condition may be predefined in the method described herein, e.g. they may be predefined and stored in a memory of the system performing the method, and said predefined weights may be set in accordance with clinical standards. In this way, the heat score of each facet may quantify an overall health status of the inspection site, while giving more weight to those dental conditions which demand particular attention by the dental practitioner and/or the dental expert.

After determining the heat score of each facet comprised in the inspection site 508, the epicenter 512a of the dental conditions 1,...,$K$ is determined in step 512 for the inspection site 508 by determining a maximum heat score of the inspection site 508. For example, the heat scores $S_1 = 10$, $S_4 = 25$, $S_8 = 9$, $S_N = 25$ may have been determined in 511 for the inspection site 508. Accordingly, the epicenter 512a of the inspection site 508 is determined to be:

$$E = \max[S_1, S_4, S_8, S_N] = S_4 = S_N$$

The epicenter 512a of the dental conditions 1,..., $K$ of the inspection site 508 comprises, in the example illustrated in Figure 5b, two facets for which a same maximum heat score is determined, i.e. the facet 505 $x_N$ and the facet 509 $x_4$. It will appear clear to those skilled in the art that the epicenter of an inspection site may comprise more than two facets, or even a single facet, depending on the number of facets comprised in the inspection site for which the determined heat score is the maximum heat score of the inspection site. Furthermore, the epicenter 512a determined in 512 is a local epicenter, i.e. the epicenter 512a is locally determined for the inspection site 508 in the example illustrated in Figure 5b. However, in another example (not illustrated here) the epicenter 512a may be a global epicenter, i.e. the epicenter 512a may be determined upon determining the global maximum heat score among the heat scores determined for all of the inspection sites defined for the 3D model 301. In other words, even though the example illustrated in Figure 5b refers to the epicenter of the inspection site 508, the method of the present disclosure may comprise determining a global epicenter, i.e. the global maximum heat score among all the heat scores determined for the data points of the 3D model.

Once the epicenter 512a is determined, in 513 the facets associated with the maximum heat score 511b and 511d are associated with a location 514 of the epicenter 512a. The latter may be used by the dental practitioner and/or by any dental expert as an accurate guide to perform the examination of the dental site. For example, the examination may be started by looking at the location of the dental site associated with the epicenter of the dental conditions, as this is determined to be the location for which the overall health status is locally the most severe, and which may therefore need careful investigation to draw up a treatment plan and/or a prevention plan for the patient.

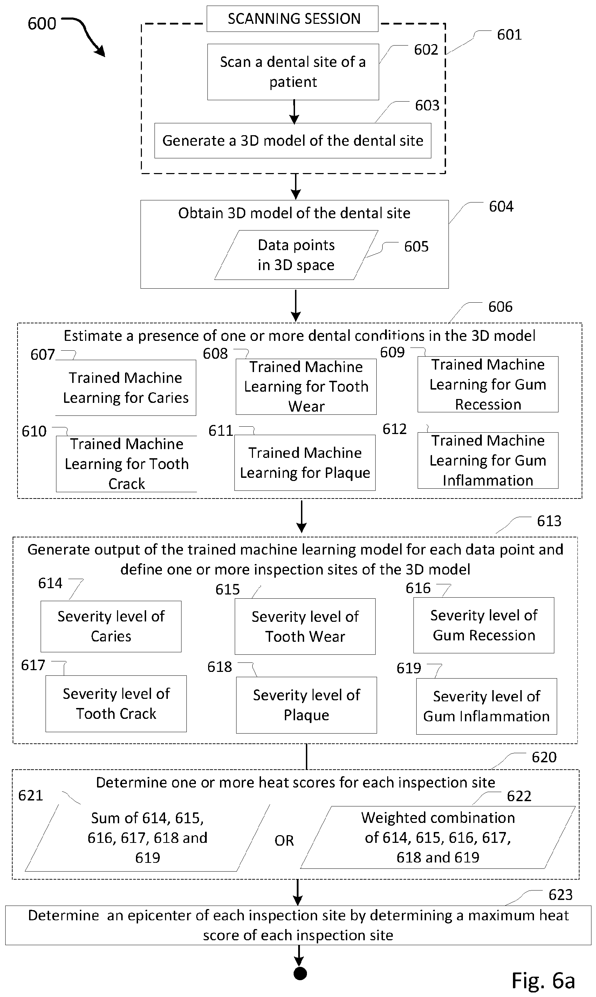

Figure 6a illustrates a flowchart 600 of an embodiment of the computer implemented method according to the present disclosure. During a scanning session 601, a dental site of a patient is scanned in step 602. The scanning may be performed by means of an intraoral scanner as the one illustrated as reference number 201 in Figure 2. Based on the data acquired during the scanning session 601, a 3D model of the scanned dental site is generated in step 603. For example, the intraoral scanner may acquire a number of 2D sub-scans for a number of viewing angles of the dental site, and thus the 3D model may be generated in step 603 by stitching together sub-scans which at least partially overlap. In one example, the scanning session 601 is carried out in a clinical visit of the patient, and therefore the 3D model is generated in step 603 during the clinical visit. In another example, the scanning session was performed in a previous clinical visit of the patient, and thus the 3D model was generated in said previous clinical visit and stored, e.g. on a memory of the computer system shown as reference number 205 in Figure 2.

The 3D model of the dental site is obtained in step 604, e.g. by loading the 3D model from the memory on which it was stored during the scanning session 601. The 3D model comprises a plurality of data points 605 collectively describing a surface of the dental site in 3D space. As also mentioned above, said plurality of data points 605 may comprise a point cloud comprising a plurality of points in 3D space, each point described by Cartesian coordinates $(x, y, z)$, or the plurality of data points 605 may comprise a plurality of facets comprised in a triangulated mesh, and/or a plurality of vertices comprised in a triangulated mesh, or any other data suitable to describe the surface of the dental site in 3D space.

In step 606, a presence of one or more dental conditions in the 3D model obtained in step 604 is estimated. The one or more dental conditions may comprise one or more of caries, tooth wear, gum recession, tooth crack, plaque, and/or gum inflammation. However, the method disclosed herein is not limited to the above mentioned dental conditions, and any other dental condition of interest for inferring a health condition of the dental site may be comprised in the one or more dental conditions.

Step 606 comprises processing the plurality of data points 605 using a trained machine learning model for each of the one or more dental conditions. Accordingly, the plurality of data points 605 is input in a trained machine learning model for caries 607, and in a trained machine learning model for tooth wear 608, and in a trained machine learning model for gum recession 609, and in a trained machine learning model for tooth crack 610, and in a trained machine learning model for plaque 611, and in a trained machine learning model for gum inflammation 612. Each of these trained machine learning models may be a pre-trained machine learning model which has been trained with a training data set comprising data points similar to the plurality of data point 605, wherein each data point in the training data set may comprise features relative to that data point in the training data set. For example, the features may be the presence or absence of a specific dental condition in a specific data point of the training data set, such that each data point in the training data set of the trained machine learning model for the specific dental condition comprises information about the presence or absence of the dental condition in that data point. Further, the features may be a severity label, and/or size, and/or area and so forth of each dental condition. For example, each point in the training machine learning model for caries 607 may comprise information about a presence or an absence of caries and/or a severity level of caries in that point, and each data point in the training data set of the trained machine learning model for tooth wear 608 may comprise information about a presence or absence of tooth wear and/or about a severity level of tooth wear in that point, and each data point in the training data set for the machine learning model for gum recession 609 may comprise information about a presence or absence of gum recession and/or about a severity level of gum recession in that point, and so forth for each of the one or more dental conditions. The presence of each dental condition may be determined using methods different than the one illustrated in the example of Figure 6a (not illustrated here), e.g. mathematical and/or statistical methods using color information comprised in the data relative to the dental site acquired during the scanning session 601.

The trained machine learning model for each dental condition used in step 606 to process the plurality of data points 605 outputs in step 613, for each of the plurality of data points 605, a severity level of each dental condition. Therefore, for each of the plurality of data points 605, a severity level of caries 614 is determined, and a severity level of tooth wear 615 is determined, and a severity level of gum recession 616 is determined, and a severity level of tooth crack 617 is determined, and a severity level of plaque 618 is determined, and a severity level of gum inflammation 619 is determined. The severity level of each dental condition may be a number on a predefined scale of severity of that dental condition, the number quantifying the severity of that dental condition. For example, the scale of severity of caries may comprise the numbers 0, 1, 3 and 9, wherein 0 indicates the absence of caries, 1 indicates initial caries, 3 indicates moderate caries and 9 indicates severe caries. The scale of severity of each dental condition may be chosen by a dental practitioner performing an examination of the dental site based on clinical features of that dental condition and/or based on a clinical significance of that dental condition. For example, the dental practitioner may decide and/or need to assign more clinical significance to caries than plaque, and accordingly the numbers on the scale of severity of caries corresponding to initial, moderate and severe caries may be chosen to be larger than the numbers on the scale of severity of plaque corresponding to initial, moderate and severe plaque, respectively. In general, the scale of severity of each dental condition comprises a minimum severity level which is indicative of an absence of the dental condition. The one or more data points in 605 for which at least one among the determined severity levels 614, 615, 616, 617, 618 and 619 is different than the minimum severity level of the scale of severity of the respective dental condition define one or more inspection sites of the 3D model. Therefore, each inspection site of the 3D model may be understood as a region of the 3D model for which the presence of at least one dental condition is determined.

For each data point comprised in each inspection site defined in step 613, an aggregated severity level/heat score is determined in step 620, thereby determining one or more heat scores for each inspection site. The heat score of each data point in step 620 may comprise the sum 621 of the severity levels 614, 615, 616, 617, 618 and 619 determined for that data point, or the heat score of each data point may comprise a weighted sum 622 of the severity levels 614, 615, 616, 617, and 618 and 619 determined for that data point. Determining the weighted sum 622 of the severity levels of each dental conditions determined for each data point may comprise determining a weight for each of the dental conditions, wherein the weight may be, for example, chosen by a dental practitioner based on a clinical significance of each dental condition. For example, the dental practitioner may decide that caries have the largest clinical significance among the one or more dental conditions, and accordingly they may assign to caries the largest weight, and the dental practitioner may decide that gum recession has the second largest clinical significance after caries, and accordingly they may assign to gum recession the second to largest weight, and so forth. As in step 620 one heat score is determined for each data point in each defined inspection site, one or more heat scores are determined for each defined inspection site, and the number of heat scores determined for each inspection site depends on the number of data points comprised in that inspection site.

In step 623, an epicenter of the one or more dental conditions is determined for each inspection site by determining a maximum heat score of each inspection site. The epicenter may be understood as a local epicenter, i.e. it may be determined by determining a local maximum for each inspection site. The epicenter of each inspection site may comprise one or more data points of the inspection site, and it indicates a region of the inspection site for which the health condition is determined to be locally the most severe, and which may therefore necessitate a careful inspection by the dental practitioner. In another example (not shown here), one epicenter of the dental conditions is determined by determining a global maximum heat score among the one or more heat scores of all of the inspection sites of the 3D model, i.e. a global epicenter is determined. The global epicenter may comprise one or more data points of the 3D model, and it indicates a region of the inspection site for which the health condition is determined to be globally the most severe, and which may therefore necessitate the highest attention by the dental practitioner.

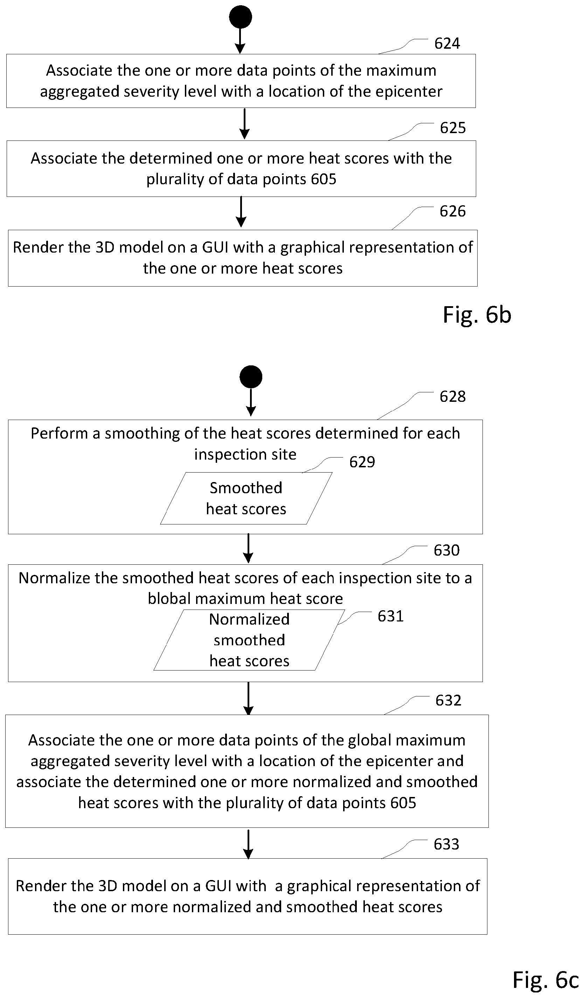

Figure 6b illustrates a flowchart of an example of additional steps of the method illustrated in Figure 6a. In step 624, the one or more data points of the maximum aggregated severity level are associated with a location of the epicenter on the dental site. Similarly, the one or more heat scores determined for each inspection site in step 620 are associated with the plurality of data point 605 of the 3D model in step 625. Associating the one or more heat scores with the plurality of data points 605 may comprise mapping each heat score to one data point of the 3D model, such that a graphical representation of the one or more heat scores may be generated. Therefore, in step 626 the 3D model of the dental site is rendered with a graphical representation of the one or more heat scores, for example on a display of the computer system as the one shown as reference number 206 in Figure 2.

The one or more heat scores may be displayed as an indication, e.g. a color or a pattern, on the rendering of the 3D model. Accordingly, the epicenter of each inspection site may be identified by the dental practitioner as the region in the graphical representation of the inspection site displayed with the most intense color or the densest pattern, and the intensity of the color or the density of the pattern may decrease in each inspection site with a distance from the epicenter of each inspection site. Here, the distance may be intended as a Euclidean distance, or a geodesic distance, or a facet distance, or any other way suitable to measure a distance between the data points comprised in the 3D model. The intensity of the color or the density of the pattern may be intended as the graphical representation of the heat score of each data point in each inspection site, such that the heat score of each inspection site is maximum at the epicenter of the inspection site, and it falls off when moving away from the epicenter, reaching a minimum value when reaching a border of the epicenter. Therefore, by looking at the rendering of the 3D model on the display of the computer system, the dental practitioner may have an accurate indication of the regions of the dental site for which the largest heat score was locally determined, and which need an accurate examination due to their determined health status.

Figure 6c illustrates a flowchart of an example of additional steps to the method illustrated in Figure 6a, alternative to the additional steps illustrated in Figure 6b. In step 628, a smoothing of the one or more heat scores determined for each inspection site is performed, thereby removing potential discontinuities between the one or more heat scores determined for neighboring points in each inspection site. The smoothing step 628 generates one or more smoothed heat scores 629 for each inspection site.

In one embodiment, the smoothing step 628 may comprise any global transformation of the heat scores determined for all of the one or more inspection sites of the 3D model. In other words, the smoothing step 628 may comprise a function depending on the epicenters of all of the inspection sites of the 3D model. For example, given a 3D model in which a number n of inspection sites has been estimated, the smoothing step 628 may comprise a function of transforming the heat scores in such a way that, given a data point $x_i$ of any of the $n$ inspection site its smoothed heat score 629 is:

$$S_{smoothed}^i = \max[\frac{E_1}{d_{i1}}, \frac{E_2}{d_{i2}}, \frac{E_3}{d_{i3}}, ..., \frac{E_n}{d_{in}}]$$

wherein $E_j$ is the heat score of the epicenter of the $j$-th (wherein $j = [1, ..., n]$) inspection site of the 3D model and $d_{ij}$ is the distance between the data point $x_i$ and the epicenter of the $j$-th inspection site. In the present example, the smoothed heat scores 629 depend on a global epicenter of the 3D model, i.e. on a global maximum heat score. In general, any function of globally transforming all the heat scores determined for the 3D model may be comprised in the smoothing step 628.

In another embodiment, the smoothing step 628 may be performed locally for each inspection site. In one example, the smoothing step 628 may comprise any function of transforming the heat scores of each inspection site such that the heat scores within the inspection site exponentially fall off with a distance from the epicenter of that inspection site. In this example, given an inspection site for which the maximum heat score $S^{Max}$ is determined, the smoothing step may comprise a function of transforming the heat scores of the inspection site such that the heat score of a data point in the inspection site at a distance d from the epicenter has a heat score of $S(d) = S^{Max}e^{-c \times d}$, where c is a constant controlling the rate at which the heat scores decrease with the distance. In another example, the smoothing step 628 may comprise any function of transforming the heat scores of each inspection site such that the heat scores of the inspection site linearly fall off with a distance from the epicenter of the inspection site. In this example, given an inspection site for which the maximum heat score $S^{Max}$ is determined, the smoothing step 628 may comprise a function of transforming the heat scores of the inspection site such that the heat score of a data point in the inspection site at a distance $d$ from the epicenter has a heat score of $S(d) = S^{Max} - c \times d$, wherein c is a constant which controls the rate at which the heat scores decrease with the distance. Here, the distance may be a Euclidean distance, a geodesic distance, a facet distance, or any other suitable way to measure a distance between two data points. In yet another example, the smoothing step 628 may comprise any function of transforming the heat scores of each inspection site such that the heat scores of the inspection site are distributed according to a Gaussian distribution centered at the value of the heat score of the epicenter of that inspection site. In this example, given an inspection site for which the maximum heat score $S^{Max}$ is determined, the smoothing step 628 may comprise a function of transforming the heat scores of the inspection site such that the heat score of a data point $x_i$ in the inspection site is:

$$S_{smoothed}\left(S_{initial}^i\right) = S^{Max} e^{-\frac{\left(S_{initial}^i - S^{Max}\right)^2}{2\,\sigma^2}}$$

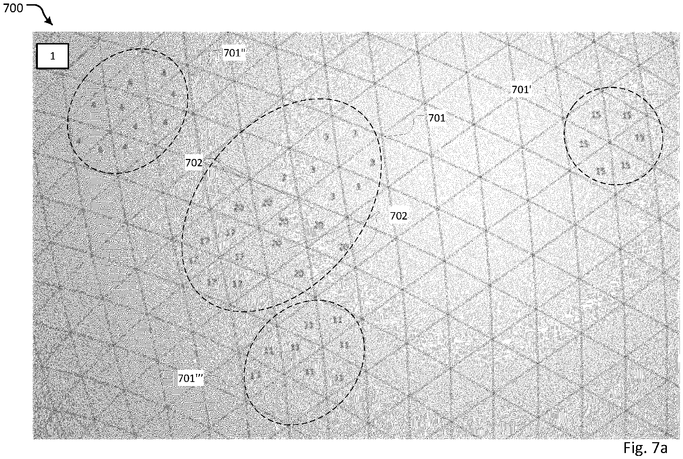

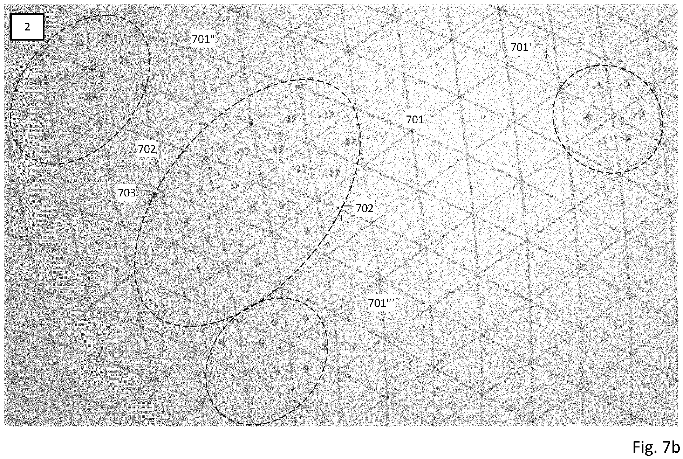

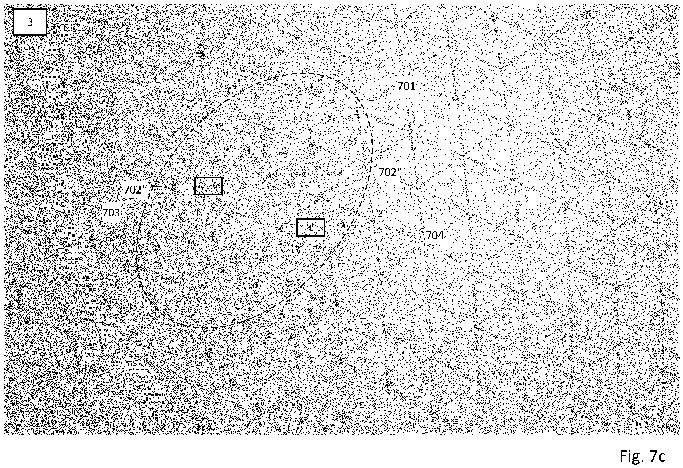

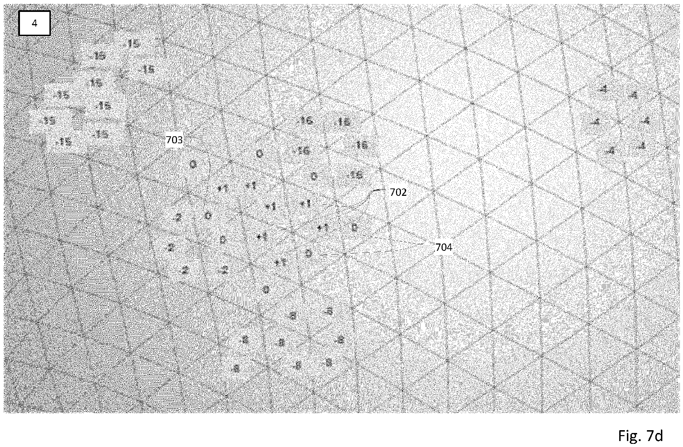

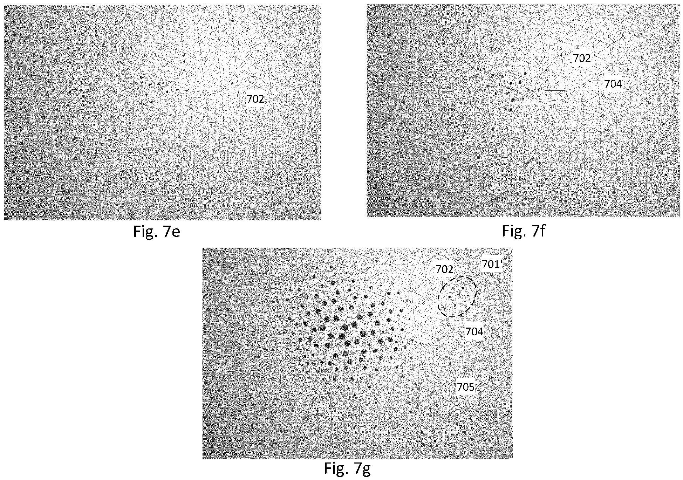

wherein $S_{initial}^i$ is the heat score initially assigned to the data point $x_i$, i.e. the heat score determined for the data point before the smoothing method 628 is performed, $\sigma = \frac{1}{N}\sum_{i=1}^{i=N}\left(S_{initial}^i - S^{Max}\right)^2$ is the variance, and $N$ is the total number of data point in the inspection site. An example of a smoothing method will be provided in the detailed description of Figure 7.

The smoothed heat scores 629 are normalized in step 630, such that one or more normalized and smoothed heat scores 631 are generated for each inspection site. Normalizing the one or more heat scores may comprise determining a global maximum heat score of the 3D model, namely determining the heat score which is the largest among all the one or more heat scores determined for all the one or more inspection sites of the 3D model. Said global maximum heat score is the global epicenter of the one or more dental conditions of the 3D model, i.e. the region of the 3D model for which the aggregated severity of the one or more dental conditions is, globally, the largest. Accordingly, the global epicenter of the 3D model corresponds to the region of the 3D model for which the overall health status is determined to be, globally, the worst. In other words, the global epicenter of the 3D model corresponds to the region of the 3D model which necessitates the highest attention. Once the global maximum heat score is determined, the one or more heat scores determined for each inspection site may be normalized to said global maximum heat score, whereby the normalized global maximum heat score is equal to 1. The normalization step also determines an order of clinical significance of the epicenters of the one or more inspection sites of the 3D model. For example, three epicenters may have been determined for three different inspection sites of the 3D model, with a smoothed heat score of 5, 10, and 3, respectively. In this example, 10 is the global maximum heat score of the 3D model, and, accordingly, the normalized smoothed heat scores of the epicenters of the 3D model are 0.5, 1, and 0.3, respectively. It appears clear that the global epicenter of the 3D model, i.e. the epicenter with a normalized smoothed heat score equal to 1, is the region with the largest clinical significance, followed by the epicenter with a normalized smoothed heat score of 0.5, and finally by the epicenter with a normalized smoothed heat score of 0.3.

In step 632, the one or more data points of the global maximum aggregated severity level are associated with a location of the epicenter on the 3D model, and, similarly, the determined one or more normalized and smoothed heat scores 631 are associated with the plurality of data points 605 of the 3D model. The mapping performed in step 632 enables to generate a graphical representation of the one or more normalized and smoothed heat scores, such that a rendering of the 3D model with said graphical representation is displayed on a GUI in step 633. The graphical representation of the one or more normalized and smoothed heat scores may be an indication, such that each inspection site is displayed with said indication on the rendering of the 3D model. The indication may be a color or a pattern, such that for each inspection site the intensity of the color or the density of the pattern decreases when moving away from the epicenter determined for that inspection site. For example, the intensity of the color or density of the pattern may decrease with a distance from the epicenter. As also mentioned above, the distance may be intended as a Euclidean distance, or as a geodesic distance, or as a facet distance, or as any other suitable way to measure a distance between the data points comprised in the 3D model. Importantly, due to the smoothing step 628 and the normalization step 629, the intensity of the color or the density of the pattern smoothly changes within each inspection site, and possible discontinuities in the color or pattern of each inspection site due to discontinuous changes of the one or more heat scores between neighboring points of the inspection site are removed by the smoothing. Therefore, the regions of the 3D model which necessitate careful examination can be clearly visualized in the rendering of the 3D model by the dental practitioner.

The intensity of the color or density of the pattern is maximum at the determined location of the global epicenter of the 3D model and progressively decreases based on the heat score of the epicenter of each inspection site relative to the global epicenter determined for the 3D model. Therefore, the determined order of clinical significance of the epicenters of the one or more inspection sites is visualized as an order of the color intensity or of the pattern density in the graphical representation of the one or more heat scores in the rendering of the 3D model. Accordingly, the dental practitioner may easily and quickly identify the region of the dental site for which the health status is determined to be globally the most severe, as this region is displayed as the most intense or as the densest on the rendering of the 3D model. Based on the intensity of the color or by the density of the pattern, the dental practitioner may perform the examination of the dental site by starting from the region with the largest intensity or densest pattern, and then proceeding to regions with progressively lower intensity or density.

Figures 7a, 7b, 7c, 7d, 7e, 7f and 7g illustrate a reference diagram 700 of an embodiment of a computer-implemented smoothing method according to the present disclosure. Illustrated in Figure 7a is an example of a processing outcome of the steps of the method according to the present disclosure. A plurality of facets is provided, wherein the plurality of facets is comprised in a triangulated mesh which may describe a surface of a dental object in 3D space. As a result of the processing, one or more of the plurality of facets have been assigned with a heat score which may be indicative of an aggregated severity level of one or more dental conditions, and which may have been determined according to the method disclosed herein. Accordingly, the outcome of the processing as illustrated in Figure 7a may have been obtained by inputting the plurality of facets in a trained machine learning model for each of the one or more dental conditions and aggregating for each facet the severity level output by the trained machined learning model for each dental condition for that facet. The one or more facets for which the heat score is different from zero, i.e. for which the presence of at least one dental condition is estimated, define one or more inspection sites of the 3D model of the dental site. With reference to Figure 7a, four inspection sites 701, 701', 701", 701''' have been defined for the triangle mesh. Each of the inspection sites comprises a local maximum heat score which is a local epicenter of the one or more dental conditions of that inspection site. For example, the epicenter of the inspection site 701 comprises the facets 702 with the heat score 20, the epicenter of the inspection site 701' comprises the facets with the heat score 15, the inspection site 701" comprises the facets with the heat score 4 and the inspection site 701''' comprises the facets with the heat score 11

Figure 7a further illustrates a first step of the smoothing method 700, in which a global maximum heat score is determined among the heat scores of the inspection sites defined for the plurality of facets. With reference to Figure 7a, the global maximum heat score among the heat scores of the inspection sites 701, 701', 701" and 701''' is 20. Accordingly, a global maximum epicenter is determined for the plurality of facets, wherein the global maximum epicenter comprises the facets 702 assigned with the global maximum heat score 20.

Figure 7b illustrates a second step of the method 700, following the step illustrated in Figure 7a. The second step comprises subtracting the global maximum heat score determined in the first step of the method from the heat score of each facet of each inspection site. Accordingly, the heat score of each facet belonging to any of the inspection sites 701, 701', 701", 701''' is reduced by 20. Thus, the facets 702 originally assigned with the global maximum heat score 20 are assigned, in this second step, with a heat score of 0, the facets 703 originally assigned with a heat score equal to 17 are assigned in this second step with a heat score equal to -3, and so forth.

Figure 7c illustrates a third step of the method 700 following the step illustrated in Figure 7b. The third step comprises determining the neighboring facets of the facets with a heat score equal to 0 which do not belong to any inspection site, i.e. which originally were not assigned with any heat score in Figure 7a. Said neighboring facets are assigned with a score of -1. For example, given the facet 702' in Figure 7c, its neighboring facets which do not belong to any inspection site are the facets 704. Accordingly, after the third step of the method 700 is performed the inspection site 701 comprises more facets than it originally did, i.e. than it did in Figure 7a. Furthermore, the third step illustrated in Figure 7b comprises determining the neighboring facets of the facets with a heat score equal to 0 which instead belong to at least one inspection site, and assigning to these facets a score of -1 only if, after the second step of the method (i.e., in Figure 7b), their score is smaller than -1. For example, given the facet 702", the neighboring facet 703 in the inspection site 701 was assigned in step two of the method with a heat score of -3, as illustrated in Figure 7b. Therefore, with reference to Figure 7c, in the third step of the method the score -1 is assigned to the facet 703.

Figure 7d illustrates the fourth step of the method 700 following the step illustrated in Figure 7c. The fourth step comprises adding a score of +1 to the score of each facet, wherein now the score is the score determined in the third step of the method (i.e., in the step illustrated in Figure 7c). Thus, referring to Figure 7d, the facets 702 are assigned with a heat score of +1, the facets 704 are assigned with a heat score of 0, the facet 703 is assigned with a heat score of zero, and so forth.

Steps three and four of the method (i.e., the steps illustrated in Figure 7c and 7d, respectively) are iteratively repeated, such that an increasing number of facets is progressively assigned with a score and a size of the inspection sites 701, 701', 701" and 701‴ progressively increases. In one example, the method may be iteratively repeated until all of the plurality of facets in the 3D model are assigned with a score. In another example, the iterations of the method may be repeated until a predefined heat score is assigned to at least one facet An example would be a predefined number of 255, in which case the smoothing step is iteratively repeated until at least one facet is assigned with a heat score of 255, i.e. it is repeated until the at least one facet of the global epicenter is assigned with the heat score of 255, and one or more facets are assigned with the minimum heat score of 0. The numbers 0 and 255 are the minimum and maximum values, respectively, of a color intensity in an 8-bit red, green, blue (RGB) system, thus in this example each facet assigned with a heat score after the smoothing step maps to a specific color intensity in the RGB system. This choice may facilitate the generation of a graphical representation of the one or more heat scores. Alternatively, the predefined number may be chosen to be a predefined percentage of the total number of facets in the plurality of facets, e.g. 30% of the total number of facets, or 60% of the total number of facets, and so on.

The iterative results of the smoothing method 700 are illustrated in Figures 7e, 7f and 7g wherein a value of the heat score is illustrated with a dot, such that the larger the heat score, the larger the size of the dot. In particular, Figure 7e illustrates a result of a first iteration of the method, i.e. the iteration illustrated in Figure 7d, wherein the facets 702 corresponding to the global maximum heat score determined in Figure 7a are assigned with a heat score equal to 1. Accordingly, after the first iteration only the facets 702 comprised in the global epicenter of the 3D model are assigned with a score and thus shown in Figure 7e.

Figure 7f illustrates a result of a second iteration of the method, i.e. the iteration following the one illustrated in Figure 7d. This iteration assigns to the facets 702 a score equal to +2, and to the facets 704 a score equal to +1. Accordingly, the facets 702 are illustrated in Figure 7f with dots larger than the ones in Figure 7d, and the neighboring facets 704 of 702 appear in Figure 7f, as they are assigned with a positive score.

The iterative method may be performed up to the iteration shown in Figure 7g wherein the score of the global maximum heat score of the inspection sites is illustrated with the largest dots among the heat scores of the remaining facets. After a number of iterations, the inspection site 701' is also visible in Figure 7g. It appears clear from Figure 7e, 7f and 7g that, overall, the smoothing method spreads the size of the inspection site 701 and ensures that the heat score smoothly changes when moving through neighboring facets.

After the smoothing method 700 is performed, the heat scores of each facet in each inspection site facets falls off with a distance from the epicenter of the corresponding inspection site, i.e. it falls off with a distance from the local maximum heat score. In one example, said distance may be a Euclidean distance. In another example, the distance from the epicenter of each inspection site may be a geodesic distance. In yet another example, the distance from the epicenter may be a facet distance. The last example may be particularly relevant in the case the computer-implemented method disclosed herein is performed upon obtaining a 3D model of the dental site which comprises a plurality of facets, such as in the case of method 700. The facet distance between a facet of an inspection site and the epicenter of that inspection site may be obtained by calculating a minimum number of steps needed to move from the epicenter to the facet, while only moving through neighboring facets. In general, the measure of the distance may be appropriately chosen based on the plurality of data points comprised in the 3D model, and the choice of this measure will appear clear to those skilled in the art, e.g. a Euclidean distance measure is more appropriate to measure the distance between points in 3D space of a point cloud.

In the example illustrated in Figure 7g, given a facet at the distance $d$ from the epicenter 702 the heat score $S$ of that facet after the smoothing method has been performed may be determined as:

$$S(d) = S^{Max} - d,$$

wherein $S^{Max}$ is the maximum heat score of the inspection site, i.e. the heat score of the epicenter of the inspection site. It will be appreciated that in the illustrated example the epicenter is actually the global epicenter of the 3D model, however the above mentioned discussion may hold for each inspection site of the 3D model. For example, if after the smoothing the facets 702 comprised in the epicenter of the inspection site are assigned with the heat score $S^{Max} = 10$, the smoothed heat score of the immediate neighboring facets 704 which are at a facet distance of $d = 1$ from the epicenter 702 is $S(1) = 9$, and the smoothed heat score of the immediate neighboring facets 705 of the facets 704 which are at a facet distance of $d = 2$ from 702 is $S(2) = 8$ and so forth.

A gap between the smoothed heat scores of facets belonging to the inspection site may be increased by elevating the heat score of each facet of the inspection site to a same predefined exponent $e$, e.g. $e = 2$, such that $S^{Max} = 100$, $S(1) = 81$, $S(2) = 64$ and so forth. The effect of this exponent is to control the rate at which the heat scores fall off when moving away from the epicenter of the inspection site, i.e. the larger the exponent, the larger the gap between the heat scores of neighboring facets and the larger the rate at which the heat score decreases when moving from the epicenter of the epicenter of the inspection site to a boundary of the inspection site. Controlling the rate at which the heat score falls off in an inspection site may be advantageous, for example, for generating a graphical representation of the heat scores and visualizing a rendering of the 3D model with said graphical representation. In this case, the exponent may be used to control the intensity of the color or the density of the pattern of the graphical representation of the heat scores, such that the intensity of the color or the density of the pattern of each inspection site falls off quickly/slowly when moving away from the epicenter of each inspection site. Advantageously, increasing the exponent e enables to emphasize the graphical representation of the epicenter of each inspection site in the rendering of the 3D model, such that a dental practitioner may easily and quickly identify the regions of the 3D model which necessitate careful examination due to their health conditions.

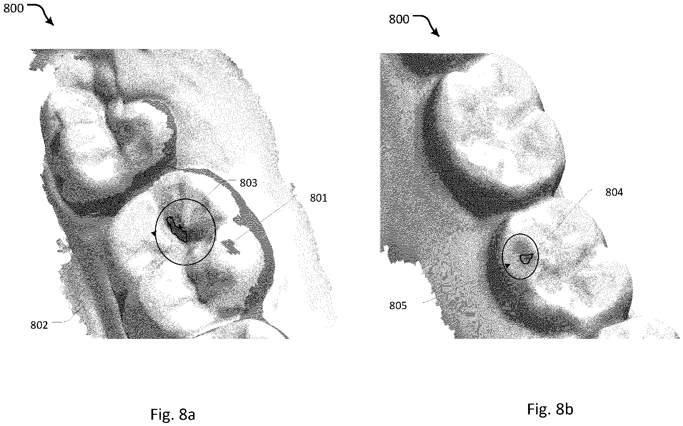

Figures 8a and 8b illustrate examples 800 of a graphical representation on a graphical-user interface (GUI) of an output of the computer-implemented method disclosed herein. The output comprises one or more aggregated severity levels determined for a 3D model of a dental site, and said 3D model comprises a plurality of data points. The graphical representation of the aggregated severity levels may be generated upon associating the determined aggregated severity levels with the plurality of data points comprised in the 3D model. In the examples illustrated in Figure 8a and 8b, the graphical representation of the aggregated severity levels is a color different than a rendering color of the 3D model. Said color may be, for example, red, or blue, or green, or any other color suitable to generate a color contrast with the rendering color of the 3D model. An intensity of the color of a point in the 3D model is determined based on the aggregated severity level associated with said point, such that the larger the intensity, the larger the aggregated severity level of the one or more dental conditions determined in that point. In another example (not shown here), the graphical representation of the aggregated severity levels may be a pattern, and the density of the pattern in a point of the 3D model may be determined based on the aggregated severity level associated with said point.

With reference to Figure 8a, regions 801 and 802 may be two inspection sites of the 3D model, i.e. a presence of at least one dental condition is estimated for each data point in 801 and 802. Accordingly, each data point in 801 and in 802 is associated with an aggregated severity level. The data points in 801 may all be associated with the same aggregated severity level, and accordingly an epicenter of the inspection site 801 comprises all its data point. An epicenter 803 of the inspection site 802 is determined to comprise only a part of the data points comprised in 802, i.e. said part of data points includes the data points of 802 associated with a maximum aggregated severity level determined for the inspection site 802. Accordingly, in the graphical representation of the inspection sites 801 and 802 the intensity of the color decreases when moving away from the epicenter of each inspection site. For example, Figure 8a illustrates as the intensity of the color decreases when moving from the epicenter 803 of the inspection site 802 to a boundary of the inspection site 802. As for the inspection site 801, a single color intensity is displayed in the graphical representation of said inspection site to indicate that all the data points comprised in 801 are comprised in the epicenter of 801. It can be noted in Figure 8a that the graphical representation of the inspection site 801 appears edgy rather than smooth, and that the color intensity discontinuously changes when moving away from the epicenter 803 of the inspection site 802. Discontinuities in the color intensity in the graphical representation of the aggregated severity levels may appear when the aggregated severity levels are not smoothed out through a smoothing method, e.g. as the one illustrated as reference number 700 in Figure 7, before generating the graphical representation.

The effect of the smoothing method on the graphical representation of the aggregated severity levels is illustrated in Figure 8b. Due to the smoothing of the aggregated severity levels, the intensity of the color of an inspection site 805 of the 3D model smoothly decreases when moving from an epicenter 804 of the inspection site 805 to a boundary of the inspection site 805. Compared to the graphical representation illustrated in Figure 8a, the graphical representation illustrated in Figure 8b clearly indicates the region(s) of the dental site which have the highest aggregated severity of the one or more dental conditions. Therefore, the graphical representation generated with the smoothed aggregated severity levels provides the dental practitioner with a more realistic and accurate indication of the regions of the dental site which necessitate careful inspection. However, it will appear clear to those skilled in the art that the graphical representation generated without smoothing the aggregated severity levels is still an effective guide for the dental practitioner to perform the examination of the dental site.

Figure 9 illustrates a rendering 900 of a 3D model of a part of a dental site with a graphical representation of aggregated severity levels of one or more dental conditions determined for a 3D model according to the present disclosure. The rendering 900 may be displayed on a display 206 of a computer system 205 on which the computer-implemented method disclosed herein is executed. A user of the computer system 205, e.g. a dental practitioner performing an examination of the dental site, may want to examine the one or more dental conditions detected in an inspection site 805 of the 3D model, and get information about the severity level of each dental condition determined for that inspection site. Accordingly, the user may input a signal which is received by the computer system 205. The input signal may comprise clicking or hovering a pointer 902 on an epicenter 804 of the inspection site 805 of the dental site, wherein the inspection site 805 is displayed on 206 as a color different than a rendering color of the 3D model. For example, the inspection site 805 may be displayed with a red color, and an intensity of the red color may be maximum at the epicenter 804 of the inspection site 805 and decrease when moving from the epicenter 804 to a boundary of the inspection site 804. The clicking or hovering may be performed by means of a mouse 211, or a keyboard 208 of the computer system, or it may be performed by means of a touchpad (not shown here) of the computer system 205. Even though the example illustrated in Figure 9 refers to a computer system 205, similar discussion holds for any system configured to perform the steps of the method described herein, and/or configured to display the rendering 900 of the 3D model according to the present disclosure For example, a tablet (not shown here) may be configured to perform the steps of the method disclosed herein, and it may be configured to display on a display the rendering 900 of the 3D model with the graphical representation of the heat scores. Alternatively, the tablet may be configured just to display the rendering 900 of the 3D model with the graphical representation of the heat scores on the display the tablet. In this case, the clicking or hovering may be performed by means of a finger touch of the tablet.

The computer system 205 may be configured to generate an ordered list of the one or more dental conditions estimated in the inspection site 804, and to store said ordered list in a memory of the computer system 205. The computer system may be further configured to load from the memory the ordered list of the inspection site 804 upon clicking or hovering on a region of the 3D model corresponding to that inspection site. With reference to Figure 9, the user may click or hover on the epicenter 804 of the inspection site 805. Accordingly, an ordered list 903 of the one or more dental conditions determined in the epicenter 804 are loaded and displayed on the display 206. The ordered list 903 may comprise the name of the detected one or more dental conditions, together with the severity level determined for each of the dental conditions. The order may be decreasing in a clinical significance of the dental conditions, such that the dental condition for which a maximum severity level is determined is displayed on the top of the ordered list 903. For example, with refence to Figure 9, in the inspection site 804 the presence of caries, tooth wear, gum recession, gum inflammation, tooth crack and plaque is determined, with a corresponding severity level of 15, 10, 4, 3, 1 and 1, respectively. Accordingly, caries are displayed on the top of the ordered list 903, followed by tooth wear, gum recession and so forth. The ordered list 903 may provide the dental practitioner with an indication of the severity of each dental condition detected in the inspection site 804, such that the dental practitioner may use the ordered list 903 as a guide to perform the examination of the dental site. For example, based on the ordered list 903, the dental practitioner may start the examination of the dental site by inspecting caries in the location associated with the inspection site 804, and then they may move to examine tooth wear, and so forth. Even though the example illustrated in Figure 9 shows only one inspection site, the above mentioned discussion holds for all of the inspection sites determined for the 3D model. This substantially enhances the diagnostic efficiency.

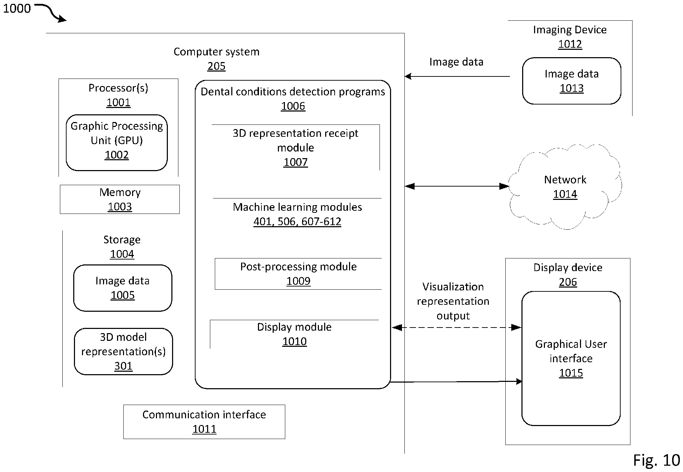

Figure 10 illustrates a system 1000 according to the present disclosure. The system 1000 may be the computer part of the dental scanning system as illustrated as reference number 200 in Figure 2. The system 1000 comprises a computer system 205, comprising a communication interface 1011 which enables the computer system 205 to exchange data with other devices. For example, the computer system 205 may be configured to receive and send data to an intraoral scanner as the one illustrated as reference number 201 in Figure 2. The computer system 205 may comprise one or more processors 1001 configured to process executable instructions, e.g. provided by a computer program. The processors 1001 may comprise a graphic processing unit 1002 and/or be configured as a central processing unit (CPU). The one or more processors 1001 may be further configured to process, partially or completely, the data received by the intraoral scanner to generate a 3D model representation(s) 301 of the dental site.

The computer system 205 further comprises a display device 206, a keyboard, touchpad, a mouse or touchscreen for entering data and activating virtual buttons (user interaction elements) visualized on the display 206. The display device 206 may be a computer screen, a touchpad screen or e.g. a smart phone screen comprising a graphical user interface 1015 and having a visual display, wherein the 3D model representation(s) 301 and e.g. a detected health condition of the dental site is displayed. The computer system 205 may comprise a memory 1003 comprising a program content executable by the processor(s) 1001, the program content comprising executable instructions to perform the computer-implemented method according to the present disclosure. Further, the computer system 205 may comprise a storage media/medium 1004 configured to store data such as the image data 1005 acquired from the intraoral scanning device during a scan session. Image data 1005 from a plurality of different intraoral scanners may be stored in storage 1004. Furthermore, image data 1005 that has been processed for using e.g. the method disclosed herein may be stored in storage 1004. Patient specific identification data, diagnostic data acquired from other scanning modalities that an intraoral scanner, and other patient relevant information may be stored in storage 1004. The storage media/medium 1004 may be configured as cloud storage or for example storage on multiple computer services which are configured to communicate with each other over a network 150. Processing and storage of data relevant for analysis by e.g. a diagnostic module may be performed in a cloud setup and loaded into a computer therefrom and/or performed locally. The storage 1004 may also store 3D model representation(s) 301 generated historically for a patient, as well as 3D model representation(s) 301 generated during a clinical visit. Furthermore, the system 1000 comprises a diagnostic module or programs 1006, each of them configured as detection program 1006 for each of one or more dental condition. Each of the diagnostic module or programs 1006 comprises executable instructions for detecting a dental condition in the 3D model representation(s) 301. The diagnostic conditions detection programs 1006 may be configured with a 3D representation receipt module 1007 for obtaining 3D model representation(s) 301 from image data 1013 of an imaging device 1012, such as the intraoral scanner illustrated as reference number 201 in Figure 2. Machine learning module 401, 506, 607-612 are configured to receive a plurality of data points comprised in the 3D model 301, wherein each trained model forming a part of each machine learning modules 401, 506, 607-612 is configured to assign severity level probabilities to each of the data points input thereto. A post-processing module 1009 is configured to perform one or more post-processing steps to the output from each of the trained learning models configured to form part of each machine learning module 401, 506, 607-612. The post-processing module may be configured to map the assigned severity level probabilities to data points of the 3D model 301. The post-processing module may be further configured to aggregate the severity level of each dental condition determined for each data point and map the aggregated severity levels to the data points of the 3D model 301. The display module 1010 may be configured to represent the 3D model 301 with a graphical representation of the determined aggregated severity levels in the graphical user interface 1015 of the display device 206.

**Items**

1. A computer-implemented method comprising the steps of:

- obtaining a three-dimensional (3D) model of a dental site, the 3D model comprising a plurality of data points describing a surface of the dental site in 3D space;
- estimating a presence of one or more dental conditions defining one or more inspection sites in the 3D model, wherein each inspection site comprises one or more of the plurality of data points;
- determining an aggregated severity level of the one or more dental conditions for each of the one or more of the plurality of data points, thereby determining one or more aggregated severity levels for each inspection site; and
- determining an epicenter of the one or more aggregated severity levels of each inspection site by determining a maximum aggregated severity level of each inspection site and associating the one or more data points of the maximum aggregated severity level with a location of the epicenter.

2. The computer-implemented method according to item 1, further comprising associating the determined one or more aggregated severity levels with the plurality of data points of the 3D model and rendering the 3D model on a graphical user interface (GUI) with a graphical representation of the one or more aggregated severity levels.

3. The computer-implemented method according to item 2, wherein the graphical representation of the one or more aggregated severity levels is displayed as an indication on the 3D model.

4. The computer-implemented method according to item 3, wherein the indication is a color different than a rendering color of the 3D model, and an intensity of the color decreases with a distance from the estimated epicenter of each inspection site.

5. The computer-implemented method according to item 3, wherein the indication is a pattern, and a density of the pattern decreases with a distance from the estimated epicenter of each inspection site.

6. The computer-implemented method according to any of items 4 or 5, further comprising calculating the distance using a Euclidean distance measure.

7. The computer-implemented method according to any of items 4 or 5, further comprising calculating the distance using a geodesic distance measure.

8. The computer-implemented method according to any of items 4 or 5, further comprising calculating the distance using a facet distance measure.

9. The computer-implemented method according to any of the previous items, wherein the one or more dental conditions comprise one or more of caries, gum recession, tooth wear, plaque, gum inflammation, and tooth crack.

10. The computer-implemented method according to any of the previous items, wherein estimating the presence of the one or more dental conditions comprises processing the plurality of data points using a trained machine learning model for each of the one or more dental conditions.

11. The computer-implemented method according to item 10, wherein the trained machine learning model for each dental condition outputs for each data point a severity level of each dental condition, and wherein the severity level is associated with a number on a predefined scale of severity of each dental condition.

12. The computer-implemented method according to item 11, wherein the predefined scale of severity of each dental condition comprises a minimum number which is indicative of an absent dental condition.

13. The computer-implemented method according to item 12, wherein the minimum number is zero.

14. The computer-implemented method according to any of the previous items, wherein determining the aggregated severity level of the one or more dental conditions for each data point comprises determining the severity level of each dental condition for each data point, and for each data point aggregating the determined severity level of each dental condition.

15. The computer-implemented method according to item 14, wherein aggregating the determined severity level of each dental condition comprises summing the determined severity level of each dental condition.

16. The computer-implemented method according to item 14, wherein aggregating the determined severity level of each dental condition further comprises determining a weighted sum of the determined severity level of each dental condition.

17. The computer-implemented method according to item 16, wherein determining the weighted sum further comprises assigning a weight to the determined severity level of each dental condition based on a clinical significance of each dental condition.

18. The computer-implemented method according to any of the previous items, wherein the plurality of data points comprises a point cloud.

19. The computer-implemented method according to any of the previous items, wherein the plurality of data points comprises a plurality of facets of a triangle mesh.

20. The computer-implemented method according to any of the previous items, wherein the plurality of data points comprises a plurality of vertices of a triangle mesh.

21. The computer-implemented method according to any of the previous items, further comprising smoothing the one or more aggregated severity levels determined for each inspection site, whereby one or more smoothed aggregate severity levels are determined for each inspection site.

22. The computer-implemented method according to item 21, wherein the one or more smoothed aggregated severity levels determined for each inspection site linearly or exponentially decrease with the distance from the epicenter determined for each inspection site.

23. The computer-implemented method according to any of the previous items, further comprising determining a global epicenter of the one or more dental conditions of the 3D model by determining a global maximum aggregated severity level among the one or more aggregated severity levels determined for the one or more inspection sites, and associating the one or more data points of the global maximum aggregated severity level with a location of the global epicenter.

24. The computer-implemented method according to any of the previous items, further comprising normalizing the one or more aggregated severity levels of the one or more inspection sites with the determined global maximum aggregated severity level, whereby the normalized one or more aggregated severity levels of the one or more inspection sites vary between 1 and 0, wherein 1 is the normalized aggregated severity level of the global epicenter of the 3D model.

25. The computer-implemented method according to any of the previous items, further comprising generating an ordered list of the one or more dental conditions estimated for each inspection site and displaying the ordered list on the GUI upon receiving an input signal.

26. The computer-implemented method according to item 25, further comprising determining an order of importance of the severity level of each of the one or more dental conditions estimated for each inspection site, wherein the order is a descending order.

27. The computer-implemented method according to any of items 25 or 26, further comprising storing the ordered list generated for each inspection site and loading the ordered list upon receiving an input signal.

28. The computer-implemented method according to any of items 25-27, wherein the input signal comprises hovering or clicking on an inspection site on the displayed 3D model with the graphical representation of the one or more aggregated severity levels.

29. The computer-implemented method according to any of items 25-28, wherein the list comprises the severity level of each of the one or more dental conditions estimated for each data point in each inspection site.

30. A computer program product comprising instructions which, when executed by a computer, cause the computer to perform the method according to any of the preceding items.

31. A non-volatile computer-readable medium comprising instructions which, when executed by a computer, cause the computer to perform the method according to any of the preceding items.

32. A system comprising:

- an intraoral scanner; and
- a computer system comprising:

  - a display;
  - a communication interface;
  - one or more processors;
  - one or more memories containing a program content executable by the one or more processors, the program content comprising executable instructions to:

    a. obtain a 3D model of a dental site, the 3D model comprising a plurality of data points collectively describing a surface of the dental site in 3D space;
    b. estimate a presence of one or more dental conditions defining one or more inspection sites in the 3D model, wherein each inspection site comprises one or more of the plurality of data points;
    c. determine an aggregated severity level of the one or more dental conditions for each of the one or more of the plurality of data points, thereby determining one or more aggregated severity levels for each inspection site; and
    d. determine an epicenter of the one or more aggregated severity levels of each inspection site by determining a maximum aggregated severity level of the inspection site and associate the one or more data points of the maximum aggregated severity level with a location of the epicenter.

33. The system according to item 32, wherein the communication interface is configured to enable the computer system to exchange data with one or more external devices and with one or more external networks.

34. The system according to item 33, wherein the one or more external networks comprise at least one cloud network.

35. The system according to item 34, wherein the at least one cloud network comprises executable instructions to process the plurality of data points using a trained machine learning model for each of the one or more dental conditions.

**Claims**

1. A computer-implemented method comprising the steps of:

   - obtaining a three-dimensional (3D) model of a dental site, the 3D model comprising a plurality of data points describing a surface of the dental site in 3D space;
   - estimating a presence of one or more dental conditions defining one or more inspection sites in the 3D model, wherein each inspection site comprises one or more of the plurality of data points;
   - determining an aggregated severity level of the one or more dental conditions for each of the one or more of the plurality of data points, thereby determining one or more aggregated severity levels for each inspection site; and
   - determining an epicenter of the one or more aggregated severity levels of each inspection site by determining a maximum aggregated severity level of each inspection site and associating the one or more data points of the maximum aggregated severity level with a location of the epicenter.

2. The computer-implemented method according to claim 1, further comprising associating the determined one or more aggregated severity levels with the plurality of data points of the 3D model and rendering the 3D model on a graphical user interface (GUI) with a graphical representation of the one or more aggregated severity levels.

3. The computer-implemented method according to claim 2, wherein the graphical representation of the one or more aggregated severity levels is displayed as an indication on the 3D model.

4. The computer-implemented method according to claim 3, wherein the indication is a color different than a rendering color of the 3D model, and an intensity of the color decreases with a distance from the estimated epicenter of each inspection site.

5. The computer-implemented method according to any of the previous claims, wherein estimating the presence of the one or more dental conditions comprises processing the plurality of data points using a trained machine learning model for each of the one or more dental conditions.

6. The computer-implemented method according to claim 5, wherein the trained machine learning model for each dental condition outputs for each data point a severity level of each dental condition, and wherein the severity level is associated with a number on a predefined scale of severity of each dental condition.

7. The computer-implemented method according to any of the previous claims, wherein determining the aggregated severity level of the one or more dental conditions for each data point comprises determining the severity level of each dental condition for each data point, and for each data point aggregating the determined severity level of each dental condition.

8. The computer-implemented method according to claim 7, wherein aggregating the determined severity level of each dental condition comprises summing the determined severity level of each dental condition.

9. The computer-implemented method according to claim 7, wherein aggregating the determined severity level of each dental condition comprises determining a weighted sum of the determined severity level of each dental condition.

10. The computer-implemented method according to claim 9, wherein determining the weighted sum further comprises assigning a weight to the determined severity level of each dental condition based on a clinical significance of each dental condition.

11. The computer-implemented method according to any of the previous claims, wherein the plurality of data points comprises a plurality of facets of a triangle mesh.

12. The computer-implemented method according to any of the previous claims, further comprising smoothing the one or more aggregated severity levels determined for each inspection site, whereby one or more smoothed aggregate severity levels are determined for each inspection site.

13. The computer-implemented method according to any of the previous claims, further comprising determining a global epicenter of the one or more dental conditions of the 3D model by determining a global maximum aggregated severity level among the one or more aggregated severity levels determined for the one or more inspection sites, and associating the one or more data points of the global maximum aggregated severity level with a location of the global epicenter.

14. The computer-implemented method according to any of the previous claims, further comprising normalizing the one or more aggregated severity levels of the one or more inspection sites with the determined global maximum aggregated severity level, whereby the normalized one or more aggregated severity levels of the one or more inspection sites vary between 1 and 0, wherein 1 is the normalized aggregated severity level of the global epicenter of the 3D model.

15. A system comprising:

   • an intraoral scanner; and
   • a computer system comprising:

      - a display;
      - a communication interface;
      - one or more processors;
      - one or more memories containing a program content executable by the one or more processors, the program content comprising executable instructions to:

         e. obtain a 3D model of a dental site, the 3D model comprising a plurality of data points collectively describing a surface of the dental site in 3D space;
         f. estimate a presence of one or more dental conditions defining one or more inspection sites in the 3D model, wherein each inspection site comprises one or more of the plurality of data points;
         g. determine an aggregated severity level of the one or more dental conditions for each of the one or more of the plurality of data points, thereby determining one or more aggregated severity levels for each inspection site; and
         h. determine an epicenter of the one or more aggregated severity levels of each inspection site by determining a maximum aggregated severity level of the inspection site and associate the one or more data points of the maximum aggregated severity level with a location of the epicenter.

100

101

Obtaining a 3D model of a dental site, the 3D model comprising a plurality of data points collectively describing a surface of the dental site in 3D space.

102

Estimating a presence of one or more dental conditions defining one or more inspection sites in the 3D model, wherein each inspection site comprises one or more of the plurality of data points.

103

Determining an aggregated severity level of the one or more dental conditions for each of the one or more of the plurality of data points, thereby determining one or more aggregated severity levels for each inspection site.

104

Determining an epicenter of the one or more aggregated severity levels of each inspection site by determining a maximum aggregated sverity level of the inspection site.

105

Associating the one or more data points of the maximum aggregated severity level determined for each inspection site with a location of the epicenter.

Fig. 1

EP 4 704 104 A1

Fig. 2

32

Fig. 3a

Fig. 3b

Fig. 4

500

$x_1$   $x_N$   301

$x_2$

$x_3$

504   501

N facets   503

$x_3$   $x_2$   $x_1$   502

$x_N$

505

506

Estimate a presence of one or more
dental conditions

ML for dental condition 1   506a

ML for dental condition 2   506b

⋮

ML for dental condition $K$   506c

502   507a   507b   507c

$x_1$   $[S_1^1, S_1^2, ..., S_1^k]$

503   507a   507b   507c

$x_2$   $[S_2^1, S_2^2, ..., S_2^k]$

504   507a   507b   507c

$x_3$   $[S_3^1, S_3^2, ..., S_3^k]$

505   ⋮

507a   507b   507c

$x_N$   $[S_N^1, S_N^2, ..., S_N^k]$

508   510   505   509

508

$x_8$   $x_N$

$x_4$

$x_1$

502

$x_1 \rightarrow [S_1^1, S_1^2, ..., S_1^k]$

$x_4 \rightarrow [S_4^1, S_4^2, ..., S_4^k]$

$x_8 \rightarrow [S_8^1, S_8^2, ..., S_8^k]$

$x_N \rightarrow [S_N^1, S_N^2, ..., S_N^k]$

Fig. 5a

Fig. 5b

600

**SCANNING SESSION** — 601

Scan a dental site of a patient — 602

↓

Generate a 3D model of the dental site — 603

↓

Obtain 3D model of the dental site — 604

Data points in 3D space — 605

↓

Estimate a presence of one or more dental conditions in the 3D model — 606

607 — Trained Machine Learning for Caries

608 — Trained Machine Learning for Tooth Wear

609 — Trained Machine Learning for Gum Recession

610 — Trained Machine Learning for Tooth Crack

611 — Trained Machine Learning for Plaque

612 — Trained Machine Learning for Gum Inflammation

↓

Generate output of the trained machine learning model for each data point and define one or more inspection sites of the 3D model — 613

614 — Severity level of Caries

615 — Severity level of Tooth Wear

616 — Severity level of Gum Recession

617 — Severity level of Tooth Crack

618 — Severity level of Plaque

619 — Severity level of Gum Inflammation

↓

Determine one or more heat scores for each inspection site — 620

621 — Sum of 614, 615, 616, 617, 618 and 619

OR

622 — Weighted combination of 614, 615, 616, 617, 618 and 619

↓

Determine an epicenter of each inspection site by determining a maximum heat score of each inspection site — 623

●

Fig. 6a

624

Associate the one or more data points of the maximum aggregated severity level with a location of the epicenter

625

Associate the determined one or more heat scores with the plurality of data points 605

626

Render the 3D model on a GUI with a graphical representation of the one or more heat scores

Fig. 6b

628

Perform a smoothing of the heat scores determined for each inspection site 629

Smoothed heat scores

630

Normalize the smoothed heat scores of each inspection site to a blobal maximum heat score

Normalized smoothed heat scores 631

632

Associate the one or more data points of the global maximum aggregated severity level with a location of the epicenter and associate the determined one or more normalized and smoothed heat scores with the plurality of data points 605

633

Render the 3D model on a GUI with a graphical representation of the one or more normalized and smoothed heat scores

Fig. 6c

Fig. 7a

Fig. 7b

Fig. 7c

Fig. 7d

Fig. 7e

Fig. 7f

Fig. 7g

Fig. 8a

Fig. 8b

List of dental
conditions

- Caries: 15
- Tooth wear: 10
- Gum recession: 4
- Gum inflammation:3
- Tooth crack:1
- Plaque:1

Fig. 9

1000

**Computer system**
**205**

**Processor(s)**
**1001**

Graphic Processing
Unit (GPU)
**1002**

**Memory**
**1003**

**Storage**
**1004**

Image data
**1005**

3D model
representation(s)
**301**

**Dental conditions detection programs**
**1006**

3D representation receipt
module
**1007**

Machine learning modules
**401, 506, 607-612**

Post-processing module
**1009**

Display module
**1010**

**Communication interface**
**1011**

Image data

**Imaging Device**
**1012**

Image data
**1013**

**Network**
**1014**

Visualization
representation
output

**Display device**
**206**

Graphical User
interface
**1015**

Fig. 10

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/147160 A1 (ALIGN TECHNOLOGY INC [US]) 7 July 2022 (2022-07-07) * paragraph [0058] - paragraph [0068] * * paragraph [0094] * * claims 1-30 * ----- | 1-15 | INV. G16H30/40 G16H50/20 G16H50/30 |
| X | US 2022/202295 A1 (ELBAZ GILAD [IL] ET AL) 30 June 2022 (2022-06-30) * paragraph [0064] * * claims 1-28 * ----- | 1-15 | |
| X | US 2024/115196 A1 (MOSHE MAAYAN [IL] ET AL) 11 April 2024 (2024-04-11) * claims 1-39 * ----- | 1-15 | |

| TECHNICAL FIELDS SEARCHED (IPC) |
|---|
| G16H |

The present search report has been drawn up for all claims

| Place of search | Date of completion of the search | Examiner |
|---|---|---|
| Munich | 4 February 2025 | Rivera Farina, P |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

 

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 24 19 7325

This annex lists the patent family members relating to the patent documents cited in  the above-mentioned European search report.
The members are as contained in the European Patent Office EDP file on
The European Patent Office is in no way liable for these particulars which are merely given for the purpose of information.

04-02-2025

| Patent document cited in search report | Publication date | Patent family member(s) | | Publication date |
|---|---|---|---|---|
| WO 2022147160 A1 | 07-07-2022 | NONE | | |
| US 2022202295 A1 | 30-06-2022 | CN | 116723789 A | 08-09-2023 |
| | | EP | 4272223 A1 | 08-11-2023 |
| | | US | 2022202295 A1 | 30-06-2022 |
| US 2024115196 A1 | 11-04-2024 | NONE | | |

EPO FORM P0459

For more details about this annex : see Official Journal of the European Patent Office, No. 12/82